# EUROPÄISCHE PATENTSCHRIFT

(11) **EP 2 795 318 B1**
(45) Veröffentlichungstag und Bekanntmachung des Hinweises auf die Patenterteilung: **20.07.2016**
(21) Anmeldenummer: 12808798.8
(22) Anmeldetag: 17.12.2012
(51) Int. Cl.: G01N 33/50, C12N 5/071

(54) **IN VITRO 3D-REPORTER-HAUTMODELL**
IN VITRO 3D-REPORTER SKIN MODEL
MODÈLE DE PEAU À GÈNE RAPPORTEUR 3D IN VITRO

(30) Priorität: 20.12.2011 DE 102011121556
(43) Veröffentlichungstag der Anmeldung: 29.10.2014
(73) Patentinhaber: Fraunhofer Gesellschaft zur Förderung der angewandten Forschung e.V., 80686 München (DE)
(72) Erfinder: BURGER-KENTISCHER, Anke, 70569 Stuttgart (DE); FINKELMEIER, Doris, 70734 Fellbach (DE); RUPP, Steffen, 70569 Stuttgart (DE); KAUFMANN, Michaela, 58332 Schwelm (DE)
(74) Vertreter: Schwahn, Hartmut
(86) Internationale Anmeldenummer: PCT/EP2012/075774
(87) Internationale Veröffentlichungsnummer: WO 2013/092480

(56) Entgegenhaltungen:
- EP-A1- 1 375 646
- WO-A1-99/45770
- WO-A2-01/92477

## Beschreibung

Die vorliegende Erfindung betrifft dreidimensionale in vitro-Hautäquivalente, deren Herstellung und Verwendung.

In den letzten Jahren haben in-vitro-Vollhautmodelle große Fortschritte gemacht. Sowohl in der pharmazeutischen Forschung als auch beim Screening potenzieller Substanzen mit therapeutischem Nutzen sind hoch entwickelte und standardisierte Testsysteme als möglichst human-nahe Indikatoren für den Wirknachweis gesucht. So wird zu diesem Zweck ein aus der EP 1 290 145 B1 bekanntes Hautmodell bereits nach DIN ISO 10993-5 für Bioverträglichkeitsprüfungen und zur Untersuchung von Chemikalien nach der neuen EU-Verordnung eingesetzt.

Das wesentliche Hauptmerkmal dieser 3D-Hautäquvivalente ist eine Kollagen-Matrix, in der primäre humane Fibroblasten eingebettet sind und so die dermale Schicht der Haut nachbilden. Auf dieser Schicht folgt eine aus humanen Keratinozyten gebildete epidermale Differenzierungsschicht mit abschließender Hornschicht. Diese Differenzierung wird während einer sogenannten Airlift-Kultivierung ausgebildet. Zudem wird durch den strukturellen Aufbau des Hautäquivalents aus dermalen und eipdermalen Zeilen eine optimale Zell-Zell-Interaktion dieser beiden Zelltypen sicher gestellt. Dadurch kann dieses Modell für unterschiedliche Fragestellungen, beispielsweise für Infektionsstudien, Wundheilungsstudien und zur Untersuchung der Melanomentstehung sowie der Wirkweise von potentiellen Medikamenten, eingesetzt werden.

Im Rahmen dieser in-vitro-Studien werden häufig sogenannte Biomarker verwendet. Diese Biomarker werden als Veränderung der Zellvitalität, morphologische Veränderungen, Änderungen im Differenzierungszustand der Epidermis, Veränderung im Genexpressionsmuster oder rein histologische Auswertungen mittels Anfärbung des Hautgewebes (z. B. Hämatoxilin/Eosin Färbung) beschrieben. Zudem dient die unterschiedliche Ausschüttung von Proteinen oder anderen zelltypischen Molekülen (z. B. Entzündungsmarker) als Biomarker, die z.B. nach Applikation einer Substanz mittels ELISA nachgewiesen werden können. Diese Nachweismethoden sind jedoch sehr zeitintensiv und komplex und erfordern aus diesem Grund auch eine weitreichende Laborausstattung und spezialisiertes Personal. Hinzu kommt, dass diese Methoden Ergebnisse liefern, welche den Ist-Zustand zum Zeitpunkt der Fixierung der Haut darstellen. Verlaufswerte können nur durch den Einsatz einer großen Vielzahl von Hautmodellen, welche zu unterschiedlichen Zeiten innerhalb des Untersuchungszeitraumes fixiert werden, erstellt werden.

Als bisherige Auswertungsparameter werden die klassischen Methoden wie der morphologische Aufbau des Hautmodells mittels Histologie und Immunhistochemie eingesetzt. Dadurch kann der Aufbau des Modells und die Ausbildung der Differenzierungsparameter (mittels Antikörper) überprüft werden. Für weitergehende Untersuchungen der Hautreaktion werden die typischen Entzündungsmarker (Zytokine), welche von Keratinozyten und Fibroblasten exprimiert werden (z.B. IL-1, IL-6, IL-8, PDGF, EOF, VEGF sowie TNF-a) im Medienüberstand mittels ELISA oder auch auf molekularbiologischer Ebene über PCR durch Probenahme detektiert. Bei vielen anderen Anwendungen müssen für den Nachweis von spezifischen Markern aber auch klassische Methoden, wie die Immunfärbung eingesetzt werden. Dafür ist eine langwierige, vorbereitende Präparation des Hautmodells erforderlich. Diese schließt sowohl die Fixierung des Modells, die Einbettung in Paraffin als auch das Anfertigen von Schnitten mit ein. An diesen Schnitten kann dann die Immundetektion der Marker mittels Antikörper erfolgen. Das schließt die Permeabilisierung des Schnittes, die Inkubation mit einen "Blockingreagenz", mehrere Waschschritte und die Inkubation mit spezifischen Antikörpern ein. Die Auswertung der Immunfärbung ist nicht normierbar oder quantifizierbar. Auch ist sie retrospektiv und Ergebnisse können nur in dem Moment der Fixierung als fester Einzelwert analysiert werden.

Aus der EP 1 375 646 A1, WO 99/45770 A1 und aus der WO 01/92477 A2 sind dreidimensionale *in vitro*-Hautäquivalente mit einer Biomatrix und darin eingebetteten Fibroblasten und Keratinozyten bekannt.

Der vorliegenden Erfindung liegt daher das technische Problem zugrunde, Hautmodelle, im Folgenden auch als in vitro-Hautäquivalente bezeichnet, insbesondere Vollhautmodelle, bereitzustellen, die die vorstehenden Nachteile überwinden, insbesondere es ermöglichen, den Einfluss von zu untersuchenden Substanzen, insbesondere auch unbekannten Substanzen, auf die Haut schnell, einfach, kostengünstig und in Echtzeit, insbesondere ohne aufwendige nachgelagerte Präparations- und/oder Fixierungstechniken, zu untersuchen. Der vorliegenden Erfindung liegt auch das technische Problem zugrunde, Verfahren bereitzustellen, die den vorgenannten Anforderungen genügen.

Die vorliegende Erfindung löst das ihr zugrundeliegende Problem durch die Bereitstellung der Lehren der unabhängigen Ansprüche 1 und 11.

Die vorliegende Erfindung löst das ihr zu Grunde liegende Problem durch die Bereitstellung eines dreidimensionalen in vitro-Hautäquivalents, umfassend eine Biomatrix und darin eingebettete Zellen mindestens eines ersten Zelltyps, dadurch gekennzeichnet, dass die Zellen des ersten Zelltyps (a) mindestens ein transgenes Reportergen, umfassend mindestens eine ein Reporterprotein codierende Nucleotidsequenz unter Kontrolle eines regulatorischen Elements, das durch mindestens einen in dem Zelltyp vorhandenen Transkriptionsfaktor induzierbar ist und (b) mindestens ein transgenes Rezeptorgen aufweisen.

Dabei löst die vorliegende Erfindung das ihr zugrundeliegende technische Problem durch die Bereitstellung eines dreidimensionalen in vitro-Hautäquivalents, umfassend eine Biomatrix und darin eingebettete Zellen mindestens eines ersten Zelltyps, wobei Zellen des ersten Zelltyps (a) ein transgenes Reportergen, umfassend mindestens eine ein Reporterprotein codierende Nucleotidsequenz unter Kontrolle eines regulatorischen Elements, das durch mindestens einen in dem Zelltyp vorhandenen Transkriptionsfaktor induzierbar ist und b) ein in vivo in dem Zelltyp vorhandenes oder transgenes Rezeptorgen aufweisen.

Das in vitro-Hautäquivalent weist Zellen mindestens eines ersten und eines zweiten Zelltyps auf.

Der erste Zelltyp ist ein Fibroblast und der zweite Zelltyp ein Keratinozyt. In besonders bevorzugter Ausführungsform sind sowohl in den Zellen des ersten als auch des zweiten Zelltyps a) jeweils mindestens ein transgenes Reportergen, umfassend jeweils mindestens eine ein Reporterprotein codierende Nucleotidsequenz unter Kontrolle eines regulatorischen Elements, das durch mindestens einen in dem Zelltyp vorhandenen Transkriptionsfaktor induzierbar ist und b) jeweils mindestens ein in vivo in dem jeweiligen Zelltyp vorhandenes oder transgenes Rezeptorgen vorhanden.

In besonders bevorzugter Ausführungsform der vorliegenden Erfindung stellen die Fibroblasten, die Keratinozyten oder beide Zellen einer, vorzugsweise humanen, Zelllinie dar. In besonders bevorzugter Ausführungsform sind die Zellen der Zelllinie immortalisierte Zellen, insbesondere sind die Fibroblasten immortalisierte primäre Fibroblasten, insbesondere immortalisierte primäre humane Fibroblasten. In besonderes bevorzugter Ausführungsform sind die Keratinozyten immortalisierte primäre Keratinozyten, insbesondere immortalisierte primäre humane Keratinozyten.

In besonders bevorzugter Ausführungsform sind die Zellen primäre Zellen, insbesondere sind die Fibroblasten, die Keratinozyten oder beide primäre Fibroblasten oder primäre Keratinozyten.

In besonders bevorzugter Ausführungsform der vorliegenden Erfindung sind die Zellen des ersten, des zweiten oder beider Zelltypen humane Zellen.

In besonders bevorzugter Ausführungsform ist vorgesehen, dass die Zellen des ersten Zelltyps, das heißt die Fibroblasten, in der Biomatrix vorwiegend oder allein eine dermale Differenzierungsschicht und die bevorzugt ebenfalls vorhandenen Zellen des zweiten Zelltyps, die Keratinozyten, in der Biomatrix vorwiegend oder allein eine über der dermalen Differenzierungsschicht liegende epidermale Differenzierungsschicht ausbilden. In besonders bevorzugter Ausführungsform ist vorgesehen, auf der epidermalen Differenzierungsschicht eine Hornschicht auszubilden. In bevorzugter Ausführungsform kann zwischen der dermalen und der epidermalen Differenzierungsschicht eine Basalmembran vorliegen.

Die vorliegende Erfindung stellt vorteilhafterweise ein dreidimensionales, in bevorzugter Ausführungsform ein humanes, in vitro-Hautäquivalent bereit, welches Zellen mindestens eines ersten Zelltyps, vorzugsweise zweier verschiedener Zelltypen, vorteilhafterweise und in bevorzugter Ausführungsform Fibroblasten und Keratinozyten, enthält, und wobei in Zellen mindestens eines dieser Zelltypen, vorteilhafterweise in Zellen beider Zelltypen, jeweils mindestens ein transgenes Reportergen vorhanden ist und wobei sich dieses mindestens eine transgene Reportergen dadurch auszeichnet, dass dieses eine ein Reporterprotein codierende Nucleotidsequenz aufweist, welche unter Kontrolle mindestens eines durch einen in den Zellen dieses Zelltyps vorkommenden Transkriptionsfaktor induzierbaren regulatorischen Elements steht und wobei die dieses transgene Reportergen aufweisenden Zellen mindestens ein transgenes und/oder ein endogen in Zellen dieses Zelltyps vorhandenes Rezeptorgen aufweisen. Sowohl Reporter- als auch Rezeptorgene sind expressionsfähig und codieren sowie exprimieren unter Kontrolle ihres regulatorischen Elementes, insbesondere Promotors, funktionsfähige Genprodukte, insbesondere Reporterproteine und Rezeptorproteine, letztere hier auch als Rezeptoren bezeichnet.

Ein derartiges dreidimensionales in vitro-Hautäquivalent ermöglicht es, den Einfluss, das heißt die Wirkung, von Substanzen, insbesondere unbekannten Substanzen, auf mindestens ein, vorzugsweise beide, Zelltypen, insbesondere auch auf das dreidimensionale in vitro-Hautäquivalent als gesamtes System, schnell, einfach, kostengünstig und in Echtzeit zu analysieren. Vorteilhafterweise ermöglichen es die Zellen des mindestens einen, ein endogen in Zellen dieses Zelltyps vorhandenes oder transgenes Rezeptorgen aufweisenden Zelltyps durch die Expression des codierten Rezeptorproteins die zu untersuchenden Substanzen, insbesondere deren Liganden, spezifisch durch Bindung an diesen Rezeptor zu erkennen und die Bindung schnell und einfach über die Expression des Reporterproteins erkennbar zu machen.

Durch die Bindung der Substanzen oder deren Liganden an den exprimierten und auf der Zelloberfläche angeordneten Rezeptor wird, vorzugsweise über eine zelleigene Signalkaskade, z. B. unter Beteiligung von in den Zellen vorhandenen Zellproteinen wie TRAF6, IKK, MyD88 und/oder IRAK, spezifisch ein diesem Rezeptor zugeordneter, in den Zellen des transfizierten Zelltyps, vorzugsweise endogen, vorkommender Transkriptionsfaktor oder mehrere Transkriptionsfaktoren aktiviert, welche(r) in Folge das in der mindestens einen ersten, vorzugsweise in beiden Zelltypen, vorhandene spezifisch durch diesen Transkriptionsfaktor induzierbare regulatorische Element eines transgenen Reportergens aktiviert. Die durch die Aktivierung induzierte Expression des transgenen Reportergens und die dadurch erfindungsgemäß ermöglichte Detektion der Induktion dieses Reportergens und damit auch der Interaktion, insbesondere Bindung, der zu analysierenden Substanz bzw. deren Liganden an einen Rezeptor, stellt eine einfache und in Echtzeit durchführbare Analysemethode für den Einfluss von Substanzen auf die Haut oder Hautmodelle dar.

Die vorliegende Erfindung stellt daher ein dreidimensionales in vitro-Hautäquivalent bereit, das eine direkte Testung von zu analysierenden Substanzen auf einem rekonstituierten dreidimensionalen Hautmodell ermöglicht und wobei über das erfindungsgemäß vorgesehene Reportergen immunmodulierende Potentiale direkt und schnell detektiert werden können. Erfindungsgemäß ist eine zeitintensive Probenvorbereitung der dreidimensionalen Hautmodelle durch Fixierung, Paraffineinbettung und Anfertigung von Schnitten, seien es Paraffin- oder auch Gefrierschnitte der zu untersuchenden Hautmodelle nicht mehr nötig. Vorteilhafterweise können die Ergebnisse der Analyse sofort erkannt werden und sind daher nicht erst zeitversetzt, insbesondere aufgrund der Fixierungspräparation, erhältlich. Darüber hinaus ist es vorteilhaft, dass es die vorliegende Verfahrensweise ermöglicht, während des Versuchs Parameter zu verändern und so einen Testablauf zu modifizieren, ohne dass eine neue Testreihe angesetzt wird.

Überraschenderweise wurde erfindungsgemäß erkannt, dass die erfindungsgemäß eingesetzten, mindestens ein transgenes Reportergen aufweisenden Zellen des ersten, vorzugsweise des ersten und zweiten, Zelltyps in einem dreidimensionalen Umfeld, das heißt einer Biomatrix, eine ligandenbindungsabhängige Expression des Reportergens und eine damit zusammenhängende zelleigene funktionsfähige Signalkaskade bereitstellen, die weitgehend dem natürlichen in vivo-Umfeld der in die dreidimensionale Matrix eingebetteten Zellen entspricht, wobei sich dieses dreidimensionale Umfeld deutlich von in Suspensionskulturen kultivierten Zellen, z. B. hinsichtlich Proliferation, Apoptose, Differenzierung und Expression, unterscheidet. Darüber hinaus unterscheiden sich primäre Zellen und etablierte Zelllinien ebenfalls deutlich voneinander z. B. hinsichtlich ihrer Verhaltensweise in Kultur, insbesondere hinsichtlich Proliferation, Apoptose, Differenzierung und Expression, was den erfindungsgemäß ermöglichten bevorzugten Einsatz von transfizierten Reportergenaufweisenden Zellen von Zelllinien des ersten und/oder zweiten Zelltyps in dreidimensionalen zusätzlich nicht-transfizierte primäre Fibroblasten aufweisenden Biomatrices besonders überraschend macht.

In bevorzugter Ausführungsform werden daher Fibroblasten, Keratinozyten oder beide, welche für die Herstellung des dreidimensionalen erfindungsgemäßen in vitro-Hautäquivalents bereitgestellt werden, zum Beispiel primäre Fibroblasten und Keratinozyten oder Zellen aus Fibroblastenzelllinien oder Keratinozytenzelllinien, zum Beispiel HaCaT, stabil mit jeweils mindestens einem spezifisch induzierbaren Reportergen transfiziert, vorzugsweise mittels eines Plasmids. Die Expression dieses mindestens einen Reportergens, welches in mindestens einem Zelltyp, vorzugsweise in beiden Zelltypen, der Biomatrix als transgenes Reportergen vorliegt, steht unter der Kontrolle mindestens eines spezifisch induzierbaren regulatorischen Elements, insbesondere eines Promotors. Dieses regulatorische Element, insbesondere Promotor, ist durch in den transfizierten endogen vorhandene Transkriptionsfaktoren über spezifische Bindestellen des regulatorischen Elements aktivierbar.

In besonders bevorzugter Ausführungsform codiert die Proteincodierende Nucleotidsequenz des Reportergens ein fluoreszierendes Protein oder ein Enzym. In besonders bevorzugter Ausführungsform ist das fluoreszierende Protein GFP, eGFP, RFP, YFP, alkalische Phosphatase (SEAP), β-galactosidase, Luciferase oder lacZ.

In einer besonders bevorzugten Ausführungsform ist vorgesehen, dass in den Zellen des ersten Zelltyps ein anderes transgenes Reportergen als in den Zellen des zweiten Zelltyps vorhanden ist, insbesondere ist vorgesehen, dass in den Zellen des ersten Zelltyps ein anderes Reporterprotein als in den Zellen des zweiten Zelltyps codiert wird.

Die Fibroblasten, Keratinozyten oder vorzugsweise Zellen beider Zelltypen, welche für die Herstellung des dreidimensionalen in vitro-Hautäquivalents bereitgestellt werden, werden darüber hinaus in optionaler, insbesondere in bevorzugter, Ausführungsform stabil mit jeweils mindestens einem ein Rezeptorprotein, auch als Rezeptor bezeichnet, codierenden Rezeptorgen stabil transfiziert, vorzugsweise mittels eines Plasmids. -

Besonders bevorzugt ist daher erfindungsgemäß ein dreidimensionales in vitro-Hautäquivalent, das mindestens ein trangenes Rezeptorgen aufweist.

Das mindestens eine transgen eingebrachte Rezeptorgen bewirkt in besonders bevorzugter Ausführungsform eine Überexpression des codierten Rezeptors oder der codierten Rezeptoren.

Die erfindungsgemäß bevorzugte Überexpression des transgen eingebrachten Rezeptors oder der Rezeptoren kann durch Auswahl geeigneter regulatorischer Elemente, insbesondere Promotoren, z. B. stark konstitutiv exprimierender Promotoren, bewirkt werden. Erfindungsgemäß bevorzugt wird beispielsweise der CMV- oder SV40-Promotor.

Die proteincodierende Nucleotidsequenz des Rezeptorgens codiert in besonders bevorzugter Ausführungsform Rezeptoren des angeborenen Immunsystems, insbesondere Rezeptoren für Immunogene, insbesondere Allergene, insbesondere PAMPs (pathogen-assoziierte molekulare Marker). In besonders bevorzugter Ausführungsform ist der Rezeptor des angeborenen Immunsystems der Pattern Recognition Rezeptor (PRR).

In einer weiteren Ausführungsform der Erfindung codiert die protein-codierende Nucleotidsequenz des Rezeptorgens einen Rezeptor für Wachstumsfaktoren oder für Zytokine, insbesondere IGF (insulin-like growth factor), FGF-2, FGF-7 (FGF: fibroblast growth factor), TNF-α (tumor necrosis factor α), TGF-α, TGF-β (transforming growth factor), EGF (epidermal growth factor), VEGF (vascular endothelial growth factor), PDGF-BB, PDGF-AB oder PDGF-β (PDGF: platelet derived growth factor).

Besonders bevorzugt sind primäre Fibroblasten, die ein Rezeptorgen für die angeborenen Immunrezeptoren TLR (Toll-like Rezeptoren) 2 und 4 aufweisen und exprimieren.

In einer weiteren bevorzugten Ausführungsform ist vorgesehen, dass für die Herstellung des Hautäquivalents Zellen eines ersten, eines zweiten oder beider Zelltypen verwendet werden, welche dadurch ausgezeichnet sind, dass diese bereits endogen in vivo mindestens ein Rezeptorgen aufweisen. In einer solchen Ausführungsform ist es nicht erforderlich, dass ein Rezeptorgen transgen in die Zellen des entsprechenden Zelltyps eingebracht wird, weil dieses bereits vorhanden ist. Der Nachweis einer Interaktion, insbesondere Bindung, mit einer zu untersuchenden Substanz erfolgt in dieser Ausführungsform über eine Interaktion mit, insbesondere eine Bindung an, den endogen in vivo vorkommenden Rezeptor. Besonders bevorzugt sind primäre Fibroblasten, die ein Rezeptorgen für die angeborenen Immunrezeptoren TLR (Toll-like Rezeptoren) 2 und 4 aufweisen und exprimieren.

In einer besonders bevorzugten Ausführungsform kann auch vorgesehen sein, dass Zellen des ersten, des zweiten oder beider Zelltypen, selbst wenn in ihnen ein endogen in vivo vorhandenes Rezeptorgen vorhanden ist, zusätzlich und vorteilhafterweise zur Signalverstärkung mit jeweils einem transgenen Rezeptorgen stabil transfiziert werden.

Der in vivo vorkommende oder transgen eingebrachte Rezeptor, z. B. PRR, wird in dem mit dem Reportergen stabil transfizierten Zelltyp, vorzugsweise in beiden Zelltypen exprimiert. Durch Interaktion mit, insbesondere durch Bindung, einer Rezeptor-bindenden Substanz, z. B. einer immunstimulierenden, Substanz, z. B. einem Allergen, wird der Rezeptor aktiviert. Durch diese Aktivierung wird in Folge, vorzugsweise über eine endogen vorhandene Signalkaskade unter Beteiligung zelleigener Proteine, eine spezifische Aktivierung von z. B. NF-κB oder anderen Transkriptionsfaktoren erreicht. In Folge der anschließenden Bindung des aktivierten Transkriptionsfaktors an das regulatorische Element des Reportergens wird eine spezifische Expression des in dem mindestens einen Zelltyp bzw. in beiden Zelltypen vorhandenen Reportergens induziert und der Nachweis der Substanz-Bindung an den Rezeptor ermöglicht.

In einer bevorzugten Ausführungsform codiert die Proteincodierende Nucleotidsequenz des Rezeptorgens Rezeptoren des angeborenen Immunsystems, insbesondere Immunogene, vorzugsweise Allergene, Rezeptoren für Wachstumsfaktoren oder Rezeptoren für Zytokine. In besonders bevorzugter Ausführungsform ist ein Rezeptor des angeborenen Immunsystems ein Mustererkennungsrezeptor, nämlich ein PRR (Pattern Recognition Receptor). In besonders bevorzugter Ausführungsform ist ein PRR ein Toll-like Rezeptor (TLR), insbesondere TLR1, TLR2, TLR3, TLR4, TLR5 oder TLR6, TLR7, TLR8, TLR9, bevorzugt TLR2 und TLR4. In einer weiteren bevorzugten Ausführungsform ist ein PRR ein C-Typ Lectin "Surfactant Protein A" (SP-A), ein Mannose-bindendes Lectin (MBL), ein Lipidtransferprotein, insbesondere ein LPS-bindendes Protein (LBP), ein Bakterien-permeabilisierendes Protein (BPI), eine zytoplasmatische Nucleotidoligomerisierungsdomäne (NOD-Protein) oder ein Scavenger-Rezeptor.

In besonders bevorzugter Ausführungsform werden Rezeptorgene eingesetzt, die vorwiegend an der Zelloberfläche immunkompotenter Zellen exprimierte PRRs codieren. In besonders bevorzugter Ausführungsform werden Rezeptorgene codierend für TLRs verwendet, die sich durch eine extrazelluläre Leucin-reiche Domäne (LRR) und eine intrazelluläre Toll/IL1-Rezeptor (TIR)-Domäne auszeichnen. Die LRRs sind verantwortlich für die Bindung der Liganden und sind charakteristisch für die TLRs, CD14 und die NOD-Proteine. Die TLR-Domäne initiiert die intrazelluläre Signaltranskription und ist homolog mit den Rezeptoren der IL-1-Familie.

In besonders bevorzugter Ausführungsform der vorliegenden Erfindung ist der PRP TLR4, der das Lipopolysaccharid (LPS) erkennt.

In besonders bevorzugter Ausführungsform ist der PRR TLR4, der den Liganden Ni²⁺ des Allergens Nickel und LPS erkennt. In besonders bevorzugter Ausführungsform ist der PRR DC-SIGN, der den Liganden Ara h 1 des Allergens Erdnussextrakt erkennt. In besonders bevorzugter Ausführungsform ist der PRR TLR2/TLR6 (Heterodimer) oder der Dectin-Rezeptor, die den Liganden Beta-1,3-Glucan des Allergens Hausstaubmilbe erkennt. In einer weiteren bevorzugten Ausführungsform ist der PRR TLR2, der das Allergen Blüten- und Gräserpollen, aber auch Bestandteile grampositiver Bakterien wie Peptidoglykane oder Lipopeptide erkennt. In besonders bevorzugter Ausführungsform ist der PRR TLR9, der das Allergen Beifuß aber auch Bakterien und Viren erkennt. In besonders bevorzugter Ausführungsform ist der PRR TSLP, der das Allergen Katzenhaar erkennt.

Die TLRs können auch als Heterodimer vorliegen, dass heißt das mindestens zwei Rezeptorprotein-codierende Bereiche stabil transfiziert und co-exprimiert werden. In einer bevorzugten Variante ist daher vorgesehen, dass die transgene Zelle zumindest zwei verschiedene TLRs co-exprimiert, so dass es zur Bildung von TLR-Heterodimern kommt, die ihre eigene Spezifität besitzen. Zu diesem Zweck weist die transgene stabil transfizierte Zelle bevorzugt ein Rezeptorgen codierend für einen ersten TOLL-like Rezeptortyp und zusätzlich ein Gen oder Gene codierend für einen zweiten TOLL-like Rezeptortyp auf.

In besonders bevorzugter Ausführungsform der vorliegenden Erfindung codiert die Protein-codierende Nukleotidsequenz des Rezeptorgens den Rezeptor MD2 (myeloid differentiation 2).

In besonders bevorzugter Ausführungsform enthalten die Zellen des ersten und gegebenenfalls des zweiten Zelltyps eine codierende Nukleotidsequenz für MD2 oder für codierende Nukleotidsequenzen von mindestens zwei Rezeptoren, zum Beispiel TLR4 und MD2.

In besonders bevorzugter Ausführungsform sind die durch den Rezeptor codierenden Bereich, insbesondere den PRR-codierenden Bereich, identifizierten Allergene PAMPs, insbesondere LPS (Lipopolysaccharide), Peptide oder Nucleinsäuren.

In besonders bevorzugter Ausführungsform wird ein dreidimensionales in vitro-Hautäquivalent bereitgestellt, umfassend eine Biomatrix und darin eingebettete Zellen mindestens einen ersten Zelltyps, insbesondere primäre oder immortalisierte Fibroblasten, insbesondere immortalisierte primäre Fibroblasten, wobei die Zellen des ersten Zelltyps (a) mindestens ein transgenes Reportergen, insbesondere zwei transgene Reportergene, jeweils umfassend mindestens eine ein Reportergen-codierende Nukeleotidsequenz unter Kontrolle eines regulatorischen Elementes aufweisen, wobei das Reporterprotein SEAP ist und das regulatorische Element, das durch mindestens einen, in dem Zelltyp vorhandenen Transkriptionsfaktor induzierbar ist, mindestens eine NF-κB-Bindestelle funktionell verbunden mit dem ELAM-Promotor ist, wobei mindestens ein transgenes Rezeptorgen, insbesondere zwei transgene Rezeptorgene, vorhanden ist beziehungsweise sind, insbesondere codierend für den TLR4 und den MD2 Rezeptor.

In besonders bevorzugter Ausführungsform ist die Biomatrix eine zur Kultivierung von Hautzellen geeignete Biomatrix, insbesondere aus polymerem Material, insbesondere biokompatiblem polymerem Material, zum Beispiel einem Protein- oder Kohlenhydratmaterial. Bevorzugt ist die Biomatrix eine dreidimensionale, gelartige Matrix aus polymerem Material, insbesondere eine dreidimensionale gelartige Kollagenmatrix. In besonders bevorzugter Ausführungsform ist die dreidimensionale gelartige Kollagenmatrix eine Matrix, wie sie in der EP 1 290 145 B1 beschrieben ist, deren Offenbarungsgehalt hinsichtlich der Herstellung und des Aufbaus dieser Kollagenmatrix einschließlich der Einbettung von Fibroblasten und Keratinozyten vollständig in den Offenbarungsgehalt dieser Lehre einbezogen wird.

Erfindungsgemäß kann die Biomatrix neben der Struktur-bildenden Komponente, insbesondere dem Polymer, weitere Komponenten, beispielsweise Wachstumsfaktoren, Adhäsionsmittel, Antibiotika, Selektionsmittel und/oder ähnliche enthalten.

Insbesondere können in der Biomatrix auch Zellen vorhanden sein, die kein transgenes Reporter- und/oder Rezeptorgen enthalten, z. B. Zellen einer Zelllinie oder primäre Zellen, vorzugsweise primäre Zellen, z. B. primäre Fibroblasten oder primäre Keratinozyten.

Unter einer "Biomatrix" wird in bevorzugter Ausführungsform eine Gelstruktur verstanden, die Kollagen, Zellkulturmedium, Serum und Puffer, beispielsweise Hepes-Puffer, enthält. In besonders bevorzugter Ausführungsform wird die dreidimensionale gelartige Kollagenmatrix hergestellt, indem aus einem Gewebe isolierte Kollagenfasern in saurer Lösung 3 bis 14 Tage bei 2 bis 10° C, vorzugsweise 4° C, gerührt, nicht gelöste Kollagenanteile abzentrifugiert und die erhaltene fertige Kollagenlösung mit einem Kollagengehalt von bevorzugt 3 mg/ml bis 8 mg/ml, vorzugsweise bei Raumtemperatur bis 37° C geliert wird.

Die Kollagenlösung, die für die Herstellung der Biomatrix verwendet wird, ist in besonders bevorzugter Ausführungsform eine Lösung, die bevorzugt einen hohen Anteil an nicht denaturiertem, nativem Kollagen in saurem, wässrigem Medium enthält, vorzugsweise mit einem pH-Wert von 3,8, beispielsweise in Essigsäure, bevorzugt in 0,1 %iger Essigsäurelösung. Ein hoher Anteil von nicht denaturiertem Kollagen bedeutet einen Anteil am Gesamtkollagen in Lösung von ≥ 50%, insbesondere ≥ 60%, ≥ 70%, ≥ 80%, ≥ 90% oder ≥ 95 %, vorzugsweise ≥ 99%. In einer bevorzugten Ausführungsform wird dabei kein lyophilisiertes Kollagen verwendet. Der Kollagengehalt der Lösung beträgt bevorzugt 3 mg Kollagen pro ml Lösung bis 8 mg Kollagen pro ml Lösung, bevorzugter 5 mg Kollagen pro ml Lösung bis 7 mg Kollagen pro ml Lösung, am bevorzugtesten 6 mg Kollagen pro ml Lösung. Vorzugsweise wird dabei Kollagen verwendet, das nach Isolierung, beispielsweise aus Rattenschwänzen, in 0,1 %iger Essigsäure drei bis vierzehn Tage bei 4° C unter Rühren inkubiert wurde und wobei nicht gelöste Kollagenanteile abzentrifugiert wurden. Bevorzugte Zellkulturmedien sind DMEM (Dulbecco's Modified Eagle Medium) und M199. Jedoch kann auch jedes andere beliebige Zellkulturmedium verwendet werden, welches die Kultivierung von Fibroblasten ermöglicht. Als Serum wird vorzugsweise fötales Kälberserum (FCS) verwendet und als Puffer zum Beispiel Hepes-Puffer. Der pH-Wert der Lösung aus Zellkulturmedium, Puffer und Serum beträgt in bevorzugter Ausführung 7,5 bis 8,5, beispielsweise 7,6 bis 8,2, insbesondere 7,8.

Im Zusammenhang mit der vorliegenden Erfindung werden unter "Substanzen", insbesondere zu untersuchenden Substanzen, Elemente, Stoffe, Verbindungen, Moleküle, Metalle, Metallkomplexe, Stoffe, Stoffverbindungen, Kristalle und biologische Systeme wie Viren, Bakterien, Mikroorganismen, Zellen oder Zellbestandteile verstanden. In bevorzugter Ausführungsform sind die Substanzen Arzneimittel, Medizinprodukte, Medizininstrumente, Zytokine, Wachstumsfaktoren, Kosmetika, Lebensmittelbestandteile, Allergene, Pyrogene, Krankheitserreger, Forschungswerkzeuge oder sollen auf ihre entsprechende Eignung hin untersucht werden.

Im Zusammenhang mit der vorliegenden Erfindung bedeutet der Begriff "Kultivieren von Zellen" ein, vorzugsweise in vitro stattfindendes, Aufrechterhalten der Lebensfunktionen von Zellen, beispielsweise Fibroblasten in einer geeigneten Umgebung, beispielsweise unter Zu- und Abfuhr von Stoffwechseledukten und -produkten, insbesondere auch eine Vermehrung der Zellen.

Im Zusammenhang mit der vorliegenden Erfindung werden unter "Fibroblasten", insbesondere dermalen Fibroblasten, natürlicherweise vorkommende, insbesondere in der Dermis vorkommende Fibroblasten, gentechnisch veränderte Fibroblasten oder deren Voläufer verstanden. Fibroblasten stellen die Vorläufer dermale Fibrozyten, das heißt spindelförmiger Zellen des Haut-Bindegewebes mit ovalem Kern und langen Fortsätzen dar. Die Fibroblasten können tierischer oder vorzugsweise humaner Herkunft sein. In einer Ausführungsform sind die Fibroblasten immortalisierte primäre Fibroblasten.

Im Zusammenhang mit der vorliegenden Erfindung werden unter "Keratinozyten" Zellen der Epidermis, die verhornendes Plattenepithel bilden können oder gentechnisch veränderte Keratinozyten und deren Vorläufer verstanden, die tierischer oder humaner Herkunft, vorzugsweise humaner Herkunft sein können. In besonders bevorzugter Ausführungsform werden undifferenzierte Keratinozyten-Stammzellen aus humanem Biopsiegewebe, das heißt Cytokeratin-19 bzw. Integrin β1-positive basale Stammzellen verwendet. In einer Ausführungsform sind die Keratinozyten immortalisierte primäre Keratinozyten.

In besonders bevorzugter Ausführungsform werden vorkultivierte Zellen, insbesondere Keratinozyten der ersten oder zweiten Zellpassage, verwendet.

Im Zusammenhang mit der vorliegenden Erfindung wird unter einem "Reportergen" ein mindestens ein induzierbares regulatorisches Element und eine funktionell damit verbundene proteincodierende Nucleotidsequenz enthaltendes Gen oder Konstrukt verstanden, das demgemäß unter Kontrolle des induzierbaren regulatorischen Elementes, insbesondere eines Promotors, steht, und dessen proteincodierende Nucleotidsequenz für ein Protein mit Reporteraktivität, insbesondere einer Fluoreszenz- oder Enzymaktivität codiert. Eine dem Reporterprotein entsprechende Aktivität, z. B. eine Enzym- oder eine Fluoreszenzaktivität, ist in der zu transfizierenden Zelle vor der Transfektion in vivo endogen nicht oder nur in unwesentlichem Maße vorhanden. Das Auftreten der von dem Reportergen, insbesondere deren codierenden Region, codierten Aktivität, z. B. Fluoreszenz- oder Enzymaktivität zeigt die Induktion, hier auch als Aktivierung bezeichnet, des regulatorischen Elementes, insbesondere des Reportergenpromotors, an.

Codiert in einer besonders bevorzugten Ausführungsform die proteincodierende Nucleotidsequenz für ein Protein mit Fluoreszenzaktivität, kann der Nachweis einer Rezeptorbindung direkt erfolgen, z. B. über eine Detektion der Fluoreszenz der Zellen im Hautmodell nach entsprechender Anregung oder über die Zugabe eines geeigneten Substrates in das Kultivierungsmedium des Hautmodells, z. B. pnPP oder BCIP. Der Nachweis ist online und/oder als Echtzeitmessung möglich. Codiert in einer anderen Ausführungsform der vorliegenden Erfindung die proteincodierende Nucleotidsequenz des Reportergens für ein Protein mit Enzymaktivität, kann in bevorzugter Ausführungsform ein spezifisches Substrat in das Kultivierungsmedium gegeben und die Expression des Reporterproteins, z. B. die alkalische Phosphatase im Medium über einen Farbumschlag photometrisch oder visuell nachgewiesen werden.

In einer bevorzugten Ausführungsform der Erfindung wird das Reportergen mit seinem induzierbaren regulatorischen Element, insbesondere Promotor, mit einem Reportergenplasmid in die Zellen transfiziert.

Die Erfindung sieht vor, das induzierbare regulatorische Element, insbesondere den induzierbaren Promotor, des Reportergens durch mindestens einen in der transfizierten endogen vorhandenen Transkriptionsfaktor zu induzieren. Im Zusammenhang mit der vorliegenden Erfindung ist ein induzierbares regulatorisches Element ein durch mindestens einen Transkriptionsfaktor induzierbares regulatorisches Element.

In einer Ausführungsform kann auch vorgesehen sein, dass, sofern in der Zelle des betreffenden Zelltyps kein geeigneter Transkriptionsfaktor endogen vorhanden ist, dieser transgen über Transfektion mit einer geeigneten Transkriptionsfaktor-codierenden Nucleotidsequenz eingebracht wird.

Gemäß der vorliegenden Erfindung werden unter dem Begriff "Transkriptionsfaktor" Moleküle verstanden, die an ein regulatorisches Element, insbesondere einen Promotor, binden und durch diese Bindung eine Induktion, das heißt Aktivierung, des Promotors mit darauffolgender Expression, das heißt Transkription und Translation, der von dem regulatorischen Element, insbesondere Promotor, kontrollierten Protein-codierenden Nucleotidsequenz erlauben.

Erfindungsgemäß sind die Zellen des mindestens einen Zelltyps, vorzugsweise beider Zelltypen, durch die Anwesenheit eines oder mehrerer der folgenden Transkriptionsfaktoren in vivo ausgezeichnet, nämlich den nucleären Faktor κB (nuclear factor kappa-lightchain-enhancer of activated B-cells) (NF-κB), ISRE (Interferon-Stimulated Response Element) 3, ISRE 5, ISRE 7, AP-1 (activator protein 1), NFAT (nuclear factor of activated T-cell), Smad (Drosophila-Gen; Mad (Mother against decapentaplegic), C. elegans Gen: Sma (Small body size), Kombination "Smad"), p53, p38/MAPK, Elk1, c-myc, c-Jun, c-Fos, STAT (signal transductors and activators of transcription), insbesondere STAT1 und STAT3.

In einer besonders bevorzugten Ausführungsform, gemäß der die proteincodierende Nukleotidsequenz des Rezeptorgens Rezeptoren des angeborenen Immunsystems, insbesondere für Immunogene, vorzugsweise für Allergene, codiert, weisen sich die Zellen des mindestens einen Zelltyps, vorzugsweise beider Zelltypen, durch die Anwesenheit mindestens eines Transkriptionsfaktors aus, ausgewählt aus der Gruppe bestehend aus NF-κB, AP-1, ISRE 3, ISRE 5, ISRE 7, NFAT und c-Fos.

In einer weiteren besonders bevorzugten Ausführungsform der vorliegenden Erfindung, gemäß der die proteincodierende Nukleotidsequenz des Rezeptorgens Rezeptoren für Wachstumsfaktoren oder Rezeptoren für Zytokine codiert, weisen sich die Zellen des mindestens einen Zelltyps, vorzugsweise beider Zelltypen, durch die Anwesenheit mindestens eines Transkriptionsfaktors aus, ausgewählt aus der Gruppe bestehend aus NF-κB, Smad, AP-1, p53, p38/MAPK, Elk1, c-myc, c-jun, c-Fos, STAT, insbesondere STAT1 und STAT3.

In besonders bevorzugter Ausführungsform kann der Nachweis von TGF-β über eine Rezeptorbindung und anschließende Aktivierung des Smad-Transkriptionsfaktors stattfinden. In einer weiteren bevorzugten Ausführungsform kann der Nachweis des Zytokins VEGF über eine Rezeptorbindung und anschließende Aktivierung des STAT-Transkriptionsfaktors stattfinden. In einer weiteren bevorzugten Ausführungsform kann der Nachweis des Zytokins PDGF-β über eine Rezeptorbindung und anschließende Aktivierung des NF-κB Transkriptionsfaktors stattfinden. In besonders bevorzugter Ausführungsform kann insbesondere die Bindung der drei vorstehend genannten aktivierten Transkriptionsfaktoren an einen induzierbaren Promotor eines transgenen Reportergens erfolgen, der die Expression des eGFP(RFP)-Reportergens kontrolliert.

In besonders bevorzugter Ausführungsform ist das mindestens eine durch mindestens einen Transkriptionsfaktor induzierbare regulatorische Element, insbesondere ein Promotor, ein Element, insbesondere Promotor, das oder der durch einen oder mehrere der genannten Transkriptionsfaktoren spezifisch induzierbar ist, insbesondere durch einen Transkriptionsfaktor ausgewählt aus der Gruppe bestehend aus nucleären Faktor κB (nuclear factor kappa-light-chain-enhancer of activated B-cells) (NF-κB), ISRE (Interferon-Stimulated Response Element) 3, ISRE 5, ISRE 7, AP-1 (activator protein 1), NFAT (nuclear factor of activated T-cell), Smad (Drosophila-Gen; Mad (Mother against decapentaplegic), C. elegans Gen: Sma (Small body size), Kombination "Smad"), p53, p38/MAPK, Elk1, c-myc, c-Jun, c-Fos und STAT (signal transductors and activators of transcription), insbesondere STAT1 und STAT3.

In besonders bevorzugter Weise ist der Promotor ein NF-κB induzierbarer Promotor. Bevorzugt ist der NF-κB induzierbare Promotor der Selektin oder ELAM-1 (endothelial cell leucocyte adhesion molecule-1) promotor.

In besonders bevorzugter Ausführungsform ist das regulatorische Element mindestens eine NF-κB-Bindestelle. In besonders bevorzugter Ausführungsform ist das mindestens eine regulatorische Element ein mindestens eine NF-κB-Bindestelle aufweisender Promotor, z.B. ELAM-Promotor, inbesondere proximaler ELAM-Promotor. In besonders bevorzugter Weise wird ein proximaler ELAM-Promotor verwendet, der Transkriptionsfaktorbindestellen aufweist, wobei eine Induzierbarkeit mittels Transkriptionsfaktoren durch die Präsenz weiterer Transkriptionsfaktorbindestellen, insbesondere NF-κB-Bindestellen, ermöglicht wird, welche vorzugsweise 5'-wärts des ELAM-Promotors angeordnet sind.

Im Zusammenhang mit der vorliegenden Erfindung sind in den Zellen des jeweiligen Zelltyps, das heißt insbesondere den Fibroblasten und Keratinozyten, die dort jeweils vorliegenden, z. B. transgen eingebrachten oder in vivo vorkommenden Reporter- oder Rezeptorgene expressionsfähig und führen in der Zelle zur Expression, das heißt Transkription und Translation der jeweilig codierten funktionalen und aktiven Genprodukte.

Im Zusammenhang mit der vorliegenden Erfindung wird unter den Begriffen "transgenes Reportergen" und "transgenes Rezeptorgen" ein Reporter- oder Rezeptorgen verstanden, das durch gentechnische Methoden in eine Zielzelle, insbesondere eine Zelle eines ersten oder zweiten Zelltyps, der vorliegenden Erfindung eingeschleust, das heißt transfiziert, wurde und dort, insbesondere in seinem Genom, stabil integriert so vorliegt, dass eine induzierbare Expression ermöglicht wird und wobei das transgene Reporter- oder Rezeptorgen endogen in vivo in der Zelle vor der Transfektion nicht vorhanden war. Derartige Gene werden auch als heterolog bezeichnet.

Im Zusammenhang mit der vorliegenden Erfindung wird unter einem "in vivo vorhandenen Rezeptorgen" ein Rezeptorgen verstanden, das in den Zellen des jeweiligen Zelltyps ursprünglich und ohne gentechnische Manipulation der Zelle bereits vorhanden ist und einen Rezeptor exprimiert. Ein derartiges Gen wird auch als endogen vorhanden bezeichnet.

Im Zusammenhang mit der vorliegenden Erfindung wird unter einem "in vitro-Hautäquivalent" sowohl ein eine epidermale und eine dermale Differenzierungsschicht aufweisendes Hautäquivalent als auch ein lediglich eine dermale oder eine epidermale Differenzierungsschicht aufweisendes Hautäquivalent, das heißt ein Dermisäquivalent oder ein Epidermisäquivalent verstanden.

Im Zusammenhang mit der vorliegenden Erfindung wird unter einem "Dermisäquivalent" eine Bindegewebe-artige Schicht aus einer Biomatrix, vorzugsweise Kollagen, und Fibroblasten verstanden, die weitgehend der nativen Dermis entspricht. Im Zusammenhang mit der vorliegenden Erfindung wird unter einem "Epidermisäquivalent" eine Schicht aus Keratinozyten und ggf. einer Biomatrix verstanden.

In besonders bevorzugter Ausführungsform ist das in vitro-Hautäquivalent ein "Vollhautmodell", das heißt ein eine epidermale und eine dermale Differenzierungsschicht aufweisendes Hautäquivalent. In einer weiteren Ausführungsform ist das in vitro-Hautäquivalent ein Dermisäquivalent, stellt also eine epidermale Differenzierungsschicht dar. In einer weiteren bevorzugten Ausführungsform ist das in vitro-Hautäquivalent ein Epidermisäquivalent, stellt also eine epidermale Differenzierungsschicht dar.

Im Zusammenhang mit der vorliegenden Erfindung wird unter "eingebetteten Zellen" verstanden, dass die Zellen in oder an der Biomatrix lokalisiert sind, das heißt entweder voll umfänglich von der Matrix umschlossen oder lediglich teilweise von der Biomatrix umfasst und teilweise frei auf dieser aufliegen, insbesondere anheften, vorzugsweise adhäsiv anheften.

In einer besonders bevorzugten Ausführungsform ist vorgesehen, dass das erfindungsgemäße dreidimensionale in vitro-Hautäquivalent, z. B. das in vitro-Vollhautmodell oder das Dermisäquivalent, zusätzlich zu den Zellen des mindestens einen ersten oder zweiten Zelltyps, vorzugsweise des ersten und zweiten Zelltyps, die sich durch die Anwesenheit jeweils mindestens eines transgenen Reportergens auszeichnen, weitere Zellen aufweist, z. B. Fibroblasten, Keratinozyten oder Fibroblasten und Keratinozyten, die kein Transgen, insbesondere kein transgenes Reportergen aufweisen. Besonders bevorzugt sind diese weiteren Zellen primäre Zellen, z. B. primäre Fibroblasten und/oder primäre Keratinozyten. Besonders bevorzugt sind die weiteren Zellen primäre Zellen, z. B. primäre Fibroblasten und/oder primäre Keratinozyten und die Zellen des ersten oder ersten und zweiten Zelltyps sind transfizierte Zellen einer Zelllinie oder immortalisierte primäre Zellen.

Die Erfindung betrifft auch Verfahren zur Herstellung des vorgenannten erfindungsgemäßen dreidimensionalen in vitro-Hautäquivalents. In besonders bevorzugter Ausführungsform sieht die Erfindung demgemäß vor, dass (a) Zellen des ersten Zelltyps und Zellen des zweiten Zelltyps bereitgestellt werden, (b) Zellen des ersten Zelltyps, mit einem transgenen Reportergen transfiziert werden, das mindestens eine ein Reporterprotein-codierende Nucleotidsequenz unter Kontrolle eines durch mindestens einen in vivo in Zellen dieses Zelltyps vorhandenen Transkriptionsfaktor induzierbaren regulatorischen Elements, insbesondere Promotors, aufweist und (c) wobei die Zellen des ersten und zweiten Zelltyps in einer dreidimensionalen Biomatrix eingebettet werden und so ein erfindungsgemäßes Hautäquivalent hergestellt wird.

Erfindungsgemäß sieht das Verfahren zur Herstellung des erfindungsgemäßen dreidimensionalen in vitro-Hautäquivalents also vor, in Verfahrensschritt (a) Zellen eines ersten und zweiten Zelltyps bereitzustellen und in Verfahrensschritt (c) die Zellen beider Zelltypen in der dreidimensionalen Biomatrix einzubetten.

In bevorzugter Ausführungsform werden Zellen des ersten und zweiten Zelltyps mit jeweils einem transgenen Reportergen transfiziert, das mindestens eine ein Rezeptorprotein codierende Nucleotidsequenz aufweist, welches unter Kontrolle eines durch einen in dem Zelltyp vorhandenen Transkriptionsfaktor induzierbaren regulatorischen Elementes steht und wobei die transfizierten Zellen beider Zelltypen in der dreidimensionalen Biomatrix eingebettet werden.

In einer Ausführungsform der vorliegenden Erfindung kann vorgesehen sein, dass Zellen des ersten, des zweiten oder beider Zelltypen bereits in vivo ein Rezeptorgen aufweisen, dessen Genprodukt in der Lage ist, einen zu untersuchenden Liganden bzw. diesen Liganden aufweisende Substanz zu binden und in Folge der Bindung zumindest einen Transkriptionsfaktor zu aktivieren. In dieser Ausführungsform ist es nicht notwendig, wenn auch möglich, ein transgenes Rezeptorgen in Zellen des ersten, des zweiten oder beider Zelltypen einzubringen.

In einer anderen besonders bevorzugten Ausführungsform kann vorgesehen sein, Zellen des ersten, des zweiten oder beider Zelltypen mit jeweils mindestens einem transgenen Rezeptorgen zu transfizieren, so dass stabil mit einem expressionsfähigen Rezeptorgen transfizierte Zellen erhalten werden.

In besonders bevorzugter Art und Weise werden daher in Verfahrensschritt (a) und (b) Zellen eines ersten, eines zweiten oder von beiden Zelltypen erhalten und bereitgestellt, die sich durch die Anwesenheit eines transgenen Reportergens und eines entweder in vivo in dem Zelltyp bereits vorhandenen oder transgenen Rezeptorgens auszeichnen.

Die Transformation der Zellen des ersten, des zweiten oder beider Zelltypen mit dem Reportergen und dem Rezeptorgen kann entweder in Form ein Co-Transfektion oder sukzessive geschehen, wobei entweder die Zellen zunächst mit dem Reportergen und anschließend dem Rezeptorgen oder umgekehrt transfiziert werden können.

In einer besonders bevorzugten Ausführungsform ist vorgesehen, dass in dem erfindungsgemäßen in vitro-Hautäquivalent zusätzlich zu den mindestens ein transgenes Reportergen aufweisenden Zel- len, z. B. des ersten Zelltyps, des zweiten Zelltyps oder des ersten und zweiten Zelltyps, weitere Zellen vorhanden sind, z. B. Fibroblasten, Keratinozyten oder beide, die nicht gentechnisch verändert wurden, insbesondere kein Transgen, insbesondere kein transgenes Reportergen aufweisen. Derartige weitere Zellen können in besonders bevorzugter Ausführungsform in Verfahrensschritt a) bereitgestellt und in Verfahrensschritt c) in die Biomatrix eingebettet werden.

In besonders bevorzugter Ausführungsform ist vorgesehen, dass die das transgene Reportergen aufweisenden Zellen, z. B. des ersten oder zweiten Zelltyps, vorzugsweise des ersten und zweiten Zelltyps, Zellen einer Zelllinie sind und dass zusätzlich zu diesen, mindestens ein transgenes Reportergen aufweisenden Zellen, Zellen ohne transgenes Reporter- und/oder ohne transgenes Rezeptorgen in der Biomatrix eingesetzt werden, vorzugsweise primäre Zellen, insbesondere primäre humane Zellen, insbesondere primäre humane Fibroblasten. In besonders bevorzugter Ausführungsform sind die vorstehend erwähnten, kein Transgen, insbesondere kein Reportergen aufweisenden, zusätzlich in dem Hautäquivalent vorhandenen Zellen primäre Zellen und die das Transgen aufweisenden Zellen sind Zel-len einer Zelllinie.

In besonders bevorzugter Ausführungsform wird daher ein Verfahren zur Herstellung eines dreidimensionalen Dermisäquivalentes bereitgestellt, wobei die vorgenannten Verfahrensschritte (a), (b) und (c) durchgeführt werden.

Die zur Kultivierung der Zellen des ersten Zelltyps, z. B. der Fibroblasten, vorgesehene Biomatrix enthält in bevorzugter Ausführungsform die zu kultivierenden transfizierten Zellen, bevorzugt Fibroblasten, und ein aus einer, vorzugsweise frischen, Kollagenlösung menschlichen oder tierischen Ursprungs neu konstituiertes Kollagengerüst einer Konzentration von bevorzugt mindestens 3 mg Kollagen pro ml Biomatrix, vorzugsweise 3,5 bis 4,5 mg Kollagen pro ml Biomatrix. Das Kollagengerüst wird bevorzugt aus einer, vorzugsweise zellfreien, sauren Lösung von Kollagen I, gewonnen, wobei die Proteinkonzentration der Kollagenlösung vorzugsweise mindestens 3 mg/ml, insbesondere 5 bis 7 mg/ml beträgt.

Die Erfindung steht daher auch in Zusammenhang mit Verfahren zur Herstellung einer dermale transfizierte Fibroblasten enthaltenden Biomatrix, also eines Dermisäquivalents, wobei in einem ersten Schritt frisches Kollagen menschlichen oder tierischen Ursprungs, beispielsweise aus Rattenschwänzen, hergestellt wird, indem aus kollagenhaltigem Gewebe isolierte Kollagenfasern in Pufferlösung gesammelt, in Alkohol oberflächlich desinfiziert und anschließend in Pufferlösung gewaschen und anschließend in eine saure Lösung eines pH-Wertes von 0,1 bis 6,9, vorzugsweise 2,0 bis 5,0, besonders bevorzugt 3,0 bis 4,0, insbesondere 3,3, zum Beispiel eine 0,1 %ige Essigsäurelösung, überführt werden. Anschließend wird in einem weiteren Schritt das in der Lösung befindliche Kollagen bei 2 bis 10° C, insbesondere 4° C, für einige Tage, zum Beispiel 3 bis 14 Tage, gerührt, die nicht gelösten Kollagenanteile werden abzentrifugiert und eine Kollagenlösung mit einem Kollagengehalt von 3 mg/ml bis 8 mg/ml bei 2 bis 10° C, zum Beispiel 4° C, erhalten.

Zur Herstellung der erfindungsgemässen Fibroblasten-haltigen Biomatrix wird bevorzugt das Material der Biomatrix, vorzugsweise also die Kollagenlösung, bei bevorzugt 2° C bis 10° C, vorzugsweise bei 4° C, mit einer Lösung, enthaltend ein, vorzugsweise fünffach konzentriertes, Zellkulturmedium, vorzugsweise fünffach konzentriertes M199-Zellkulturmedium, Puffer, vorzugsweise Hepes-Puffer, Serum, vorzugsweise fötales Kälberserum (FCS), und Chondroitin- (4/6)-sulfat, mit den transfizierten Fibroblasten, vorzugsweise 1 bis 2 x 10⁵/ml transfizierte Fibroblasten, insbesondere vorkultivierten Fibroblasten, versetzt und gemischt. Dieses Gemisch wird bevorzugt in Kulturgefäße gegeben und durch Erhöhung der Temperatur auf beispielsweise Raumtemperatur oder 37° C erfolgt eine Gelierung. Nach dem Gelieren der Fibroblasten-Kollagengele wird in bevorzugter Ausführungsform Fibronectin, vorzugsweise humanes Fibronectin, auf die Gele gegeben. Durch die Zugabe von Fibronektinen zur Fibroblasten-Kollagenmatrix wird die Bindung der Fibroblasten sowohl an Kollagen als auch untereinander begünstigt. Die anschließende Kultivierung der Fibroblasten im Kollagengel erfolgt vorzugsweise in Submers-Kultur.

Im Zusammenhang mit der vorliegenden Erfindung wird unter einer "Submers-Kultivierung" oder einer "Submers-Kultur" ein Verfahren zur Kultivierung von Zellen verstanden, wobei die Zellen mit einer Nährlösung bedeckt sind. Die Fibroblasten enthaltende Biomatrix wird in bevorzugter Ausführungsform mit Zellkulturmedium überschichtet und bei 35 bis 39° C, insbesondere 37° C inkubiert.

In einer vorteilhaften Ausführungsform werden die dermalen transfizierten Fibroblasten in der dreidimensionalen Biomatrix, wie vorstehend beschrieben, kultiviert, so dass ein Dermisäquivalent, hier auch als dermale Differenzierungsschicht bezeichnet, gewonnen werden kann.

Das so erhaltene erfindungsgemäße Dermisäquivalent kann für Analyseverfahren für Substanzen, z. B. für Screening- und Diagnoseverfahren verwendet werden, insbesondere zur Untersuchung der Wirkungen chemischer Substanzen, beispielsweise potentieller Arzneimittel oder Bestandteile von Kosmetika.

Die Erfindung steht auch mit Analyseverfahren in Zusammenhang, insbesondere Screening- und Diagnoseverfahren, unter Verwendung der erfindungsgemäßen Dermisäquivalente.

Eine bevorzugte Ausführung der Erfindung betrifft ein Verfahren zur Analyse des Dermisäquivalents in An- und Abwesenheit der zu untersuchenden Substanz und den Vergleich der beobachteten Auswirkungen auf die Zellen oder Zellbestandteile des Dermisäquivalents.

In besonders bevorzugter Ausführung wird das so hergestellte Dermisäquivalent verwendet, um darauf in einem zweiten Verfahrensschritt eine epidermale Differenzierungsschicht, hier auch als Epidermisäquivalent, bezeichnet aufzubringen.

In einer bevorzugten Ausführung ist also vorgesehen, die Einbettung der Zellen gemäß Verfahrensschritt (c) in eine Biomatrix so durchzuführen, dass eine, vorzugsweise einen ersten Zelltyp, zum Beispiel die transfizierten Fibroblasten aufweisende, dermale Differenzierungsschicht und darüber liegend eine, vorzugsweise den zweiten Zelltyp, zum Beispiel die vorzugsweise transfizierten oder nicht transfizierten Keratinozyten, enthaltende epidermale Differenzierungsschicht ausgebildet wird. In Verfahrensschritt (c) wird in einer besonders bevorzugten Ausführungsform vorgesehen, in einem Verfahrensschritt (c1) die mit dem Reportergen, vorzugsweise zusätzlich mit einem Rezeptorgen, transfizierten Zellen des ersten Zelltyps in Material der Biomatrix einzubringen, dort zu kultivieren und so eine dermale Differenzierungsschicht auszubilden und in einem zweiten Verfahrensschritt (c2) auf die so ausgebildete dermale Differenzierungsschicht Zellen des zweiten Zelltyps, insbesondere die vorzugsweise transfizierten Zellen des zweiten Zelltyps, auszusäen und so eine epidermale Differenzierungsschicht zu erhalten.

Eine besonders bevorzugte Ausführung betrifft also auch ein Verfahren zur Herstellung eines aus Dermisäquivalent und Epidermisäquivalent bestehenden Hautäquivalents. Dabei werden in bevorzugter Ausführungsform ein bis drei Tage, vorzugsweise zwei Tage, nach der vorstehend beschriebenen Herstellung und Inkubation des Dermisäquivalents die vorzugsweise transfizierten Keratinocyten auf dem Dermisäquivalent ausgesät.

Die Aussaat der vorzugsweise transfizierte Keratinocyten auf die Biomatrix erfolgt vorzugsweise in einem Zellkulturmedium, besonders bevorzugt in KBM-Medium (Clonetics), das 5% fötales Kälberserum enthält. Anschliessend wird in bevorzugter Ausführungsform die Biomatrix mit KBM-Medium, enthaltend humanen epidermalen Wachstumsfaktor (hEGF) (0,1 ug/500 ml Medium), BPE (15 mg/ 500 ml Medium) und 0,8 mM CaCl₂, überschichtet und einer vorzugsweise 1-bis 3-tägigen Submers-Kultivierung unterworfen. Eine vollständige Differenzierung der Keratinocytenschichten wird in bevorzugter Ausführungsform durch eine Airlift-Kultur mit 1,8 mM CaCl₂ enthaltendem KBM-Medium ohne hEGF und BPE erreicht.

Im Zusammenhang mit der vorliegenden Erfindung wird unter einer "Airlift-Kultur" eine Kultur verstanden, wobei die Höhe des Nährmedienspiegels genau auf die Höhe der Biomatrix abgestimmt ist, während die Keratinocyten oder durch die Keratinocyten gebildeten Zellschichten über dem Nährmedienspiegel liegen und vom Nährmedium nicht bedeckt werden, das heißt die Kultivierung erfolgt an der Grenzschicht Luft/Nährmedium, wobei die Versorgung der Kulturen von unten her erfolgt. Dazu werden die Inserts aus der Mikrotiterplatte mit 24 Vertiefungen in die Vertiefungen einer Mikrotiterplatte mit 6 Vertiefungen mit jeweils einem Durchmesser von 3,5 cm übertragen. Nach einer, vorzugsweise 12-bis 14-tägigen, Airlift-Kultur entwickelt sich ein hauttypisches, Dermisäquivalent und Epidermisäquivalent umfassendes in vitro-Vollhautmodell.

Das erfindungsgemäße Verfahren zur Herstellung eines in vitro-Vollhautmodells kann vorteilhafterweise so modifiziert werden, dass vor, während oder nach der Aussaat von Keratinocyten weitere Hautzelltypen, wie Melanocyten, Immunzellen und/oder Endothelzellen, in die Biomatrix eingebracht oder auf der Biomatrix ausgesät und weiter kultiviert werden.

Die Erfindung betrifft daher auch ein hauttypisches, in vitro-Vollhautmodell, hier auch als Hautäquivalent bezeichnet, insbesondere ein humanes in vitro-Vollhautmodell, das nach dem erfindungsgemäßen Verfahren und einem gegebenenfalls anschließenden und/oder vorausgehenden Kultivierungsverfahren herkömmlicher Art hergestellt wurde. In bevorzugter Ausführungsform weist das Hautäquivalent mindestens 2 bis 4 proliferative, einige differenzierende und mindestens 4 bis 5 verhornte Zellschichten auf, wobei das Epidermisäquivalent Stratum basale, Stratum spinosum, Stratum granulo sum und Stratum corneum umfasst und wobei zwischen dem Dermisäquivalent und dem Epidermisäquivalent eine funktionsfähige Basalmembran aus Matrixproteinen enthalten ist und wobei darüber hinaus hauttypische Proteine wie Fillgrin, Ki-67 und Cytokeratin 10 exprimiert werden.

Die Erfindung betrifft demgemäß auch Verfahren zur Analyse von Substanzen, wobei die untersuchenden Substanzen mit einem Hautäquivalent der vorliegenden Erfindung, insbesondere einem dreidimensionalen in vitro-Hautäquivalent, enthaltend eine dermale und eine epidermale Differenzierungsschicht oder einem Hautäquivalent enthaltend lediglich eine dermale Differenzierungsschicht in Kontakt gebracht, optional inkubiert, und deren Wirkung auf das Hautäquivalent analysiert wird. In bevorzugter Ausführungsform wird ein Kontakt, insbesondere ein physischer Kontakt, der zu untersuchenden Substanz zu den an der Oberfläche der das Hautäquivalent ausbildenden, mit dem mindestens einen Reportergen transfizierten Zellen herbeigeführt, z. B. mit Substanzen in einer Flüssigkeit oder in Form eines Kontaktes des Hautäquivalents zu einem festen Prüfstück. In besonders bevorzugter Ausführungsform wird die Analyse durch Nachweis der Reportergenaktivität durchgeführt z. B. mittels enzymatischer oder fluoreszenzspektroskopischer Methoden. In besonders bevorzugter Ausführungsform stellt die Analyse eine Detektion der von dem induzierten Reportergen vermittelten Reporterproteinaktivität, insbesondere Enzymaktivität dar, wobei die Detektion der Reporterproteinaktivität das Vorhandensein eines an den Rezeptor bindenden Liganden anzeigt.

In besonders bevorzugter Ausführungform betrifft die vorliegende Erfindung ein Verfahren zur Analyse von mindestens einer Substanz, wobei die zu untersuchende Substanz, insbesondere ein Ligand dieser Substanz, mit einem Hautäquivalent der vorliegenden Erfindung in Kontakt gebracht und deren Wirkung auf das Hautäquivalent analysiert wird. In besonders bevorzugter Ausführungsform ist die Analyse eine Detektion der von dem mindestens einen Reportergen vermittelten Reporterproteinaktivität.

In besonders bevorzugter Ausführungsform betrifft die vorliegende Erfindung daher ein Verfahen zur Analyse von mindestens einer Substanz, wobei die zu untersuchende Substanz, vorzugsweise enthaltend mindestens einen Liganden, mit einem Hautäquivalent der vorliegenden Erfindung in Kontakt gebracht und deren Wirkung auf das Hautäquivalent analysiert wird, wobei die mindestens eine Substanz mit dem transgen eingebrachten, vorzugsweise überexprimierten, Rezeptorprotein an der Oberfläche der Zellen des Hautäquivalents so in Kontakt gebracht wird, dass eine Bindung stattfindet und eine zelleigene Signaltransduktion von dem durch die Bindung aktivierten Rezeptorprotein oder Rezeptorproteinen zu einem zelleigenen Transkriptionsfaktor stattfindet und wobei dieser Transkriptionsfaktor anmindestens eine Transkriptionsfaktorbindestelle eines transgen eingebrachten Reportergenkonstruktes bindet und eine Expression des funktionell damit verbundenen proteincodierenden Bereichs des Reportergens induziert und wobei durch die Expression des Reportergens ein Reporterprotein mittels z. B. herkömmlicher Nachweisverfahren detektiert werden kann.

In besonders bevorzugter Ausführungsform sind die erfindungsgemäß bereitgestellten Verfahren zur Analyse von mindestens einer Substanz.Verfahren zum Nachweis einer Bindung einer zu untersuchenden Substanz oder eines Liganden davon an ein Hautäquivalent, insbesondere an eine Zelle eines Hautäquivalents, geeignet.

In einer weiteren bevorzugten Ausführungsform stellt die vorliegende Erfindung ein Verfahren zum Nachweis der Bindung mindestens einer Substanz an mindestens einen transgen eingebrachten, insbesondere überexprimierten, Rezeptor eines Hautäquivalents gemäß der vorliegenden Erfindung bereit, wobei das Verfahren vorsieht, die mindestens eine zu untersuchende Substanz mit einem Hautäquvialent gemäß der vorliegenden Erfindung in Kontakt zu bringen und deren Wirkung auf das Hautäquivalent zu analysieren, wobei die Bindung der mindestens einen zu untersuchenden Substanz an den wenigstens einen Rezeptor zur Aktivierung einer zelleigenen Signaltransduktionskette führt, welche mindestens einen zelleigenen oder transgen eingebrachten Transkriptionsfaktor induziert, welcher an mindestens ein regulatorisches Element mindestens eines transgen eingebrachten Reportergens, umfassend mindestens eine ein Reporterprotein-codierende Nukleotidsequenz unter Kontrolle des regulatorischen Elementes, bindet und so eine Induktion und Expression des Reporterproteins bewirkt, welche zu einer nachweisbaren Aktivität des Reporterproteins und anschließenden Detektion der Aktivität führt.

In besonders bevorzugter Ausführungsform stellt die Erfindung ein Verfahren zur Analyse von mindestens eine Substanz, vorzugsweise von Substanzen, zur Verfügung, wobei die zu untersuchende Substanz mit einem Hautäquivalent gemäß der vorliegenden Erfindung in Kontakt gebracht und deren Wirkung auf das Hautäquivalent analysiert wird, wobei die Analyse eine Detektion der von mindestens einem induzierten Reportergen vermittelten Reporterproteinaktivität ist und die Reporterproteinaktivität die Bindung einer Substanz oder eines Liganden davon an den mindestens einen, vorzugsweise überexprimierten, Rezeptor anzeigt, inbesondere die Bindung eines an den vom Rezeptorgen codierten Rezeptor bindenden Liganden der Substanz anzeigt.

Das erfindungsgemäße dreidimensionale Hautmodell kann gezielt für Analysen der chemisch-pharmazeutischen Industrie, der Lebensmittelindustrie und der kosmetischen Industrie verwendet werden.

Besonders eignet sich das erfindungsgemäße Hautäquivalent zur Analyse von potentiellen Antagonisten, insbesondere Substanzen, welche die Toll-like Rezeptoren blockieren. Derartige Substanzen finden Einsatz in der Dermatologie. Darüber hinaus kann das erfindungsgemäße dreidimensionale Hautäquivalent zum Wirkstoff-Screening, insbesondere neuer TLR-Antagonisten eingesetzt werden.

Insbesondere eignet sich das erfindungsgemäß hergestellte Hautäquivalent zur Analyse von Produkten, das heißt zur Produktprüfung, beispielsweise auf Wirksamkeit, unerwünschte Nebenwirkungen, beispielsweise Reiz-, Toxizitäts- und Entzündungswirkungen oder allergieauslösende Wirkungen oder Verträglichkeit von Substanzen.

Eine bevorzugte Ausführungsform der Erfindung umfasst daher Verfahren zur Analyse der Wirkung, insbesondere der pharmakologischen Wirkung, von Substanzen auf Haut, insbesondere menschliche Haut, unter Verwendung des erfindungsgemäß hergestellten humanen Hautäquivalentes.

In einer besonders bevorzugten Ausführungsform der Erfindung werden die Wirkungen von Substanzen auf spezielle Hauttypen untersucht. Dabei werden Zellen definierter Hauttypen, beispielsweise Hauttypen mit wenig Pigmenten und/oder Hauttypen mit vielen Pigmenten, zur Etablierung erfindungsgemäßer Hautäquivalente eingesetzt und diese werden im Hinblick auf die Wirkung von Substanzen getestet.

In einer weiteren besonders bevorzugten Ausführungsform der Erfindung wird das erfindungsgemäß hergestellte Hautäquivalent als Modellsystem zu Analysen von Hautkrankheiten und zur Entwicklung neuer Behandlungsmöglichkeiten für Hautleiden verwendet. Beispielsweise können Zelllinien von Patienten mit einer bestimmten Hautkrankheit verwendet werden, um daraus patientenspezifische Hautmodellsysteme zu etablieren und daran die Wirksamkeit bestimmter Therapien und/oder Medikamente zu untersuchen und, optional, zu beurteilen.

Weitere Ausgestaltungen der Erfindung ergeben sich aus den Unteransprüchen.

Die Erfindung wird anhand der folgenden Beispiele und der dazugehörigen Figuren näher erläutert.

Die Figuren zeigen:
Figur 1 eine schematische Darstellung des Herstellverfahrens für das erfindungsgemäße dreidimensionale in vitro-Hautäquivalent,
Figur 2 mikroskopische Aufnahmen nicht induzierter (A) und mit LPS induzierter (B) transfizierter Fibroblasten, die ein induzierbares Fluoreszenzreportergen enthalten,
Figur 3 eine schematische Darstellung einer erfindungsgemäß ermöglichten Identifizierung von Zytokinen mittels des dreidimensionalen in vitro-Hautäquivalents,
Figur 4 mikroskopische Aufnahmen nicht induzierter (A) und mit TNF-α induzierter (β) HEK293-Zellen mit stabil transfizierten eGFP-Reportergenen,
Figur 5 eine Darstellung der spezifischen Expression von SEAP in stabil transfizierten TNF-α induzierten oder nicht-induzierten HEK293-Zellen,
Figur 6 einen Paraffinschnitt einer eGFP transfizierten Fibroblasten-Suspensionszelllinie NIH3T3A in einem dreidimensionalen Hautmodell (Isotypkontrolle: (A)), (Anti-eGFP Antikörper:(B)),
Figur 7 einen Paraffinschnitt primärer eGFP transfizierter Fibroblasten in einem dreidimensionalen Hautmodell (Isotypkontrolle (A)), (Anti-eGFP Antikörper:(B)), (A1/B1: mit Farbkamera aufgenommen, A2/B2: mit Schwarz-Weiß-Kamera aufgenommen),
Figur 8 eine schematische Darstellung eines Herstellverfahrens für das erfindungsgemäße dreidimensionale in vitro-Hautäquivalent unter Einsatz eines transgenen SEAP-Reportergens und eines transgenen TLR4/ MD2 Rezeptorkomplexes,
Figur 9 die Ergebnisse einer spezifischen Induktion primärer Fibroblastenreporterzellen, mit und ohne Rezeptorkomplex (TLR4/MD2) und
Figur 10 den Aufbau eines dreidimensionalen Hautmodels mit im-mortalisierten humanen Fibroblasten im Vergleich zu primären Fibroblasten.

### Beispiel 1 - Herstellung eines in vitro-Hautäquivalents mit transgenem Rezeptor und Rezeptorgenen

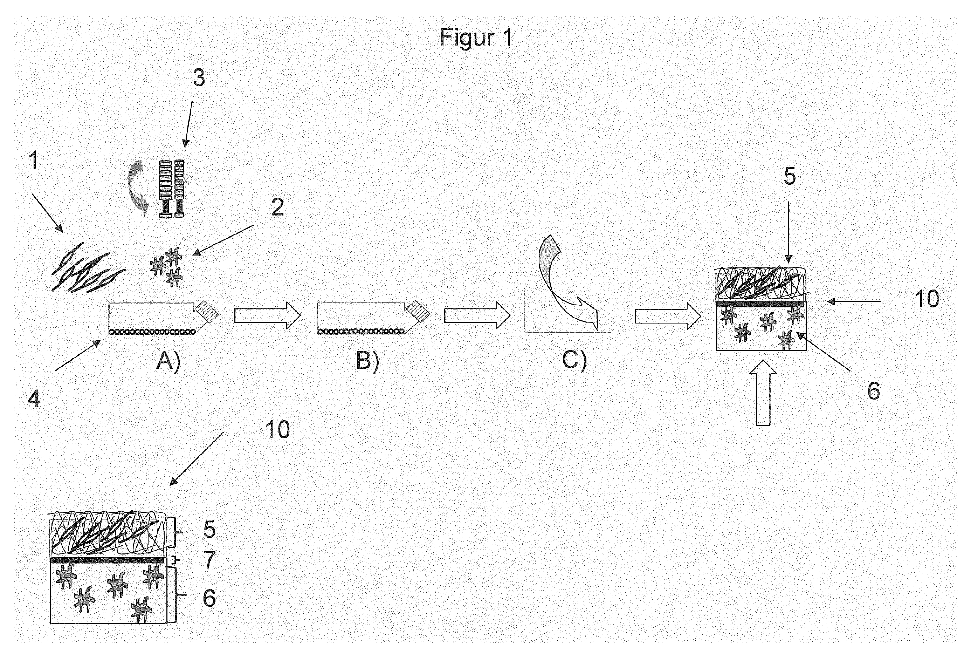

Das erfindungsgemäße dreidimensionale in vitro-Hautäquivalent wird wie folgt und wie in Figur 1 schematisch dargestellt hergestellt.

In einem ersten Verfahrensschritt A) werden primäre humane Keratinozyten (1) sowie primäre humane Fibroblasten (2) sowohl mit einem transgenen Rezeptorgen (3), dessen codierende Nukleotidsequenz einen pattern-recognition-receptor (PRR) codiert, als auch mit einem transgenen Reportergen (4), welches spezifisch über einen operativ verbundenen Promotor mit einer Bindestelle für einen induzierenden Transkriptionsfaktor induzierbar ist, transfiziert. In einem anschließenden Verfahrensschritt B) findet eine Selektion stabil transfizierter Zellen statt, also von Fibroblasten und Keratinozyten, die jeweils beide Transgene stabil enthalten und anschließend eine Lagerung der erhaltenen Zelllinien als Master Cell Bank in Stickstoff. In einem weiteren Verfahrensschritt C) wird aus diesen Zelllinien in an sich bekannter Weise, so wie beispielsweise in der EP 1 290 145 B1 beschrieben ist, ein dreidimensionales in vitro-Hautmodell (10) aufgebaut. Dies geschieht, indem zunächst eine dermale Schicht (6) mit den stabil transfizierten Fibroblasten (2) und anschließend darauf eine epidermale Schicht (5) mit den stabil transfizierten Keratinozyten (1) hergestellt und so ein dreidimensionales Hautäquivalent (10) aufgebaut wird, welches zwischen dermaler Schicht (6) und epidermaler Schicht (5) eine Basalmembran (7) (Fibronectin) enthält.

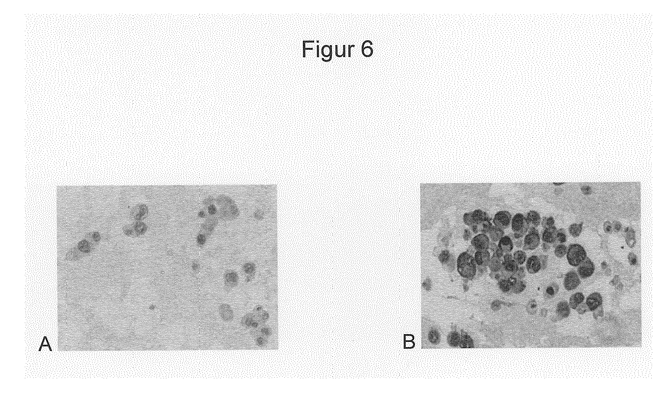

Figur 6 zeigt einen Paraffinschnitt von Zellen der Fibroblasten-Suspensionszelllinie NIH3T3A in einem dreidimensionalen Hautmodell, wobei in den Zellen eine stabile Integration und Expression eines eGFP-codierenden Reportergens erfolgte und diese über Antikörpernachweis im Mikroskop nachweisbar ist (Isotypkontrolle (A) und eGFP-Aktivität durch Antikörperfärbung nachgewiesen in (B)).

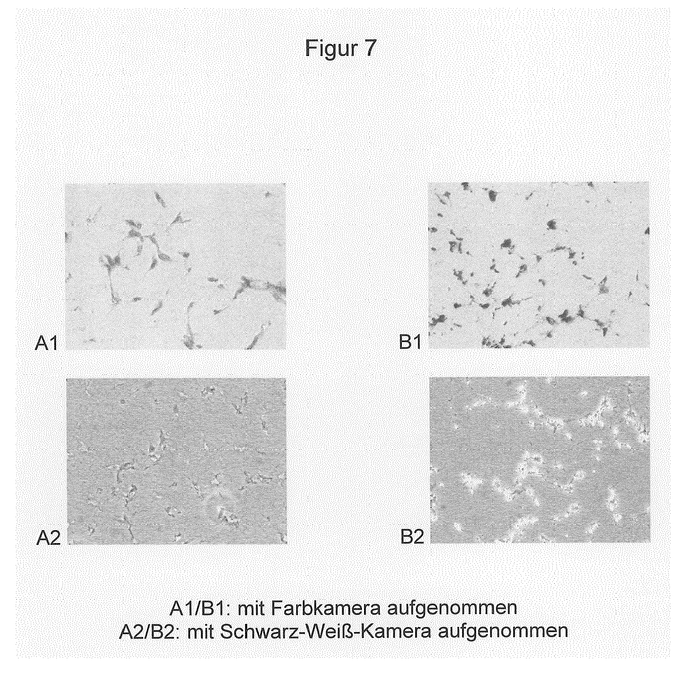

Figur 7 zeigt einen Paraffinschnitt von primären Fibroblasten in einem dreidimensionalen Hautmodell, wobei in den Zellen eine stabile Integration und Expression eines eGFP-codierenden Reportergens erfolgte und über Antikörpernachweis im Mikroskop nachweisbar ist (Isotypkontrolle (A1/A2) und eGFP-Aktivität durch Antikörperfärbung nachgewiesen in (B1/B2)).

### Beispiel 2 - Identifizierung von Allergenen

Das gemäß Beispiel 1 hergestellte Hautmodell 10 wird zur Testung der Substanzen, z. B. des allergenen Potentials von Substanzen, mit diesen in Kontakt gebracht.

Die zu testenden Substanzen können sowohl in Flüssigkeiten als auch als Bestandteil von Festkörpern, z. B. Wundauflagen, mit dem erfindungsgemäßen dreidimensionalen Hautmodell 10 in Kontakt gebracht werden.

Sollte die zu testende Substanz ein Allergen darstellen oder eine zu testende Mischung ein solches Allergen enthalten, führt dies zu einer Signalkaskade. Demgemäß wird durch Bindung des Allergens (PAMP) an den vom transgenen Rezeptorgen codierten PRR eine Aktivierung desselben ausgelöst, die zu einer Signalfolge über die endogen vorhandenen Faktoren MyD88, IRAK, TRAF6 und IKK führt, im Rahmen derer der endogen vorhandene Transkriptionsfaktor NF-κB aktiviert, anschließend ein durch diesen Transkriptionsfaktor spezifisch induzierbarer Promotor eines Reportergens aktiviert, der zur Expression von in das Zellkulturmedium sezernierten SEAP führt, so dass im Zellkulturmedium eine Phosphataseaktivität nachweisbar ist. Die Zugabe eines Substrates für die Phosphatase führt zu einem visuellen Nachweis des Allergens durch Farbumschlag oder ist nachweisbar durch Photometer.

In einem weiteren Teilaspekt einer alternativen Ausführungsform der vorliegenden Erfindung wird das Allergen LPS durch Bindung an die PRRs TLR4/CD14, eine NF-κB-Aktivierung, eine dadurch bewirkte Aktivierung eines NF-κB induzierbaren Promotors und eine dadurch induzierte SEAP-Expression nachgewiesen.

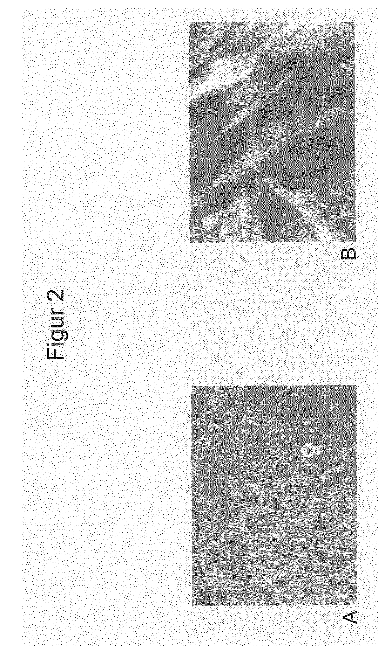

Figur 2 zeigt die Ergebnisse einer erfindungsgemäßen Ausführungsform, gemäß der primäre Fibroblasten (dermales Hautäquivalent) stabil mit NF-κB induzierbaren eGFP- und RFP-Reportergenen transfiziert wurden. Die Fibroblasten wurden darüber hinaus stabil mit Rezeptorgenen transfiziert, welche die PRRs TLR2/6 und TLR4/CD14 exprimierten. Die Induktion mit LPS in einer Mikrotiterplatte führte, wie sich aus Figur 2A (Kontrollfibroblasten ohne Induktion mit LPS) im Vergleich mit Figur 2B (LPS-induzierte Fibroblasten) ergibt, zu einem Nachweis des LPS in den stabil transfizierten primären Fibroblasten.

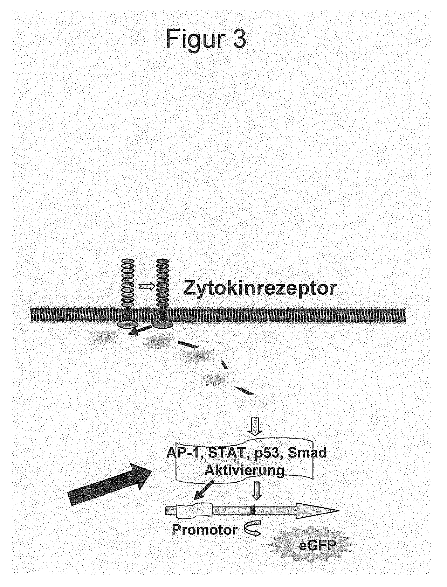

Figur 3 zeigt schematisch den Einsatz des erfindungsgemäßen in vitro-Hautäquivalents zum Nachweis von Zytokinen, wobei ein transgen codierter Zytokin-Rezeptor durch Bindung an Zytokine aktiviert, seinerseits zur Aktivierung eines endogen vorhandenen Transkriptionsfaktors führt, welcher an einen durch den Transkriptionsfaktor spezifisch induzierbaren Promotor bindet, diesen aktiviert und zur Expression von eGFP führt.

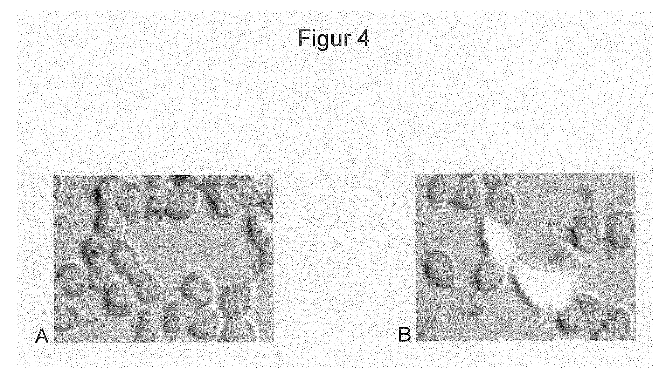

Figur 4 zeigt Ergebnisse einer erfindungsgemäßen Ausführungsform, gemäß der HEK293-Zellen stabil mit AP-1 induzierbaren eGFP- und RFP-Reportergenen transfiziert wurden. Die HEK293-Zellen wurden darüber hinaus stabil mit einem Rezeptorgen transfiziert, welches den TNF-α-Rezeptor exprimiert. Die Induktion mit TNF-α in einer Mikrotiterplatte führte, wie sich aus Figur 6A (HEK293-Kontrollzellen ohne Induktion mit TNF-α) im Vergleich mit Figur 6B (TNF-α induzierte HEK293-Zellen) ergibt, zu einem mikroskopischen eGFP-Nachweis des TNF-α in den stabil transfizierten HEK293-Zellen.

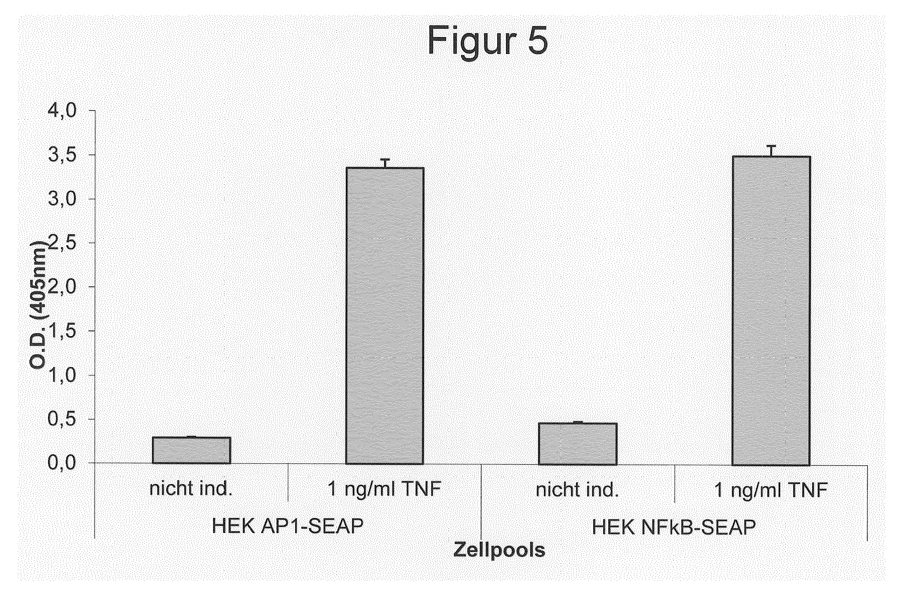

Figur 5 zeigt die Ergebnisse einer erfindungsgemäßen Ausführungsform, gemäß der HEK293-Zellen stabil mit AP-1 induzierbaren SEAP-Reportergenen transfiziert wurden. Die HEK293-Zellen wurden darüber hinaus stabil mit einem Rezeptorgen transfiziert, welches den TNF-α-Rezeptor exprimiert. Die Induktion mit TNF-α in einer Mikrotiterplatte führte, wie sich aus Figur 5 ergibt, zu einem enzymatischen Nachweis des TNF-α in den stabil transfizierten HEK293-Zellen. Figur 5 zeigt in Form eines Histogramms nichtinduzierte und mit TNF-induzierte Zellen für zwei unterschiedliche SEAP-Reportergenkonstrukte, nämlich HEK PAP1 SEAP und HEK NF-κB-SEAP.

Die Induktion mittels TNF wird durch eine signifikant gesteigerte Phosphatase-Aktivität nachgewiesen.

### Beispiel 3 Herstellung eines in vitro -Hautäquivalents mit transgenen Rezeptor- und Reportergenen

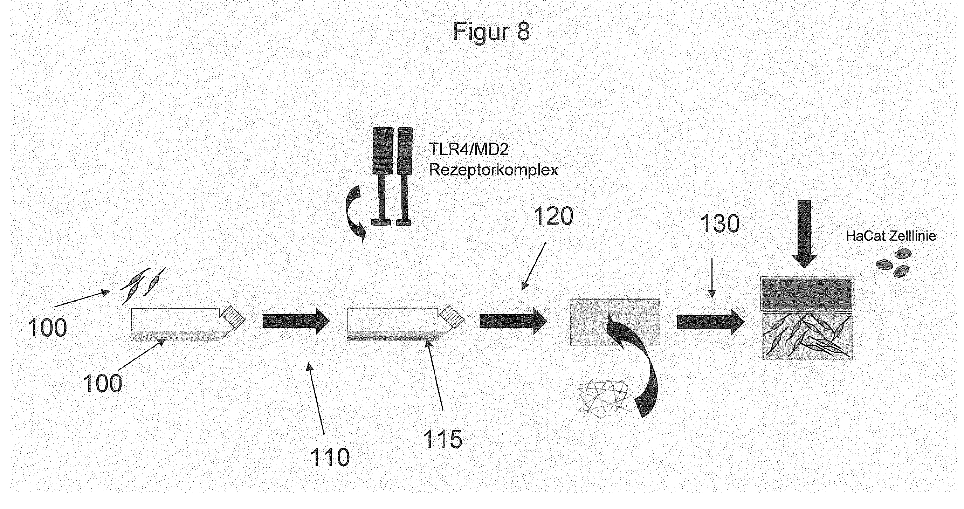

Ein erfindungsgemäßes dreidimensionales in vitro-Hautäquivalent wird wie folgt und wie in Figur 8 schematisch dargestellt hergestellt.

Zunächst wird ein Reportergenplasmid enthaltend die proteincodierende Sequenz für SEAP hergestellt, wobei dieses neben dem operativ mit den reportercodierenden Nukleotidsequenzen verbundenen ELAM-Promotor fünf zusätzliche Bindestellen für einen induzierbaren Transkriptionsfaktor (NF-κB) enthält. Der eingesetzte ELAM-Promotor ist der proximale ELAM-Promotor mit einigen Nf-κB Bindestellen, der durch zusätzliche fünf NF-κB Bindestelle induzierbar wird und so die Expression des Reportergens in Abhängigkeit der Bindung von NF-κB-Transkriptionsfaktoren aktivieren kann.

Darüber hinaus wird ein Plasmid enthaltend zwei transgene Rezeptorgene, nämlich eines codierend für TLR4 und eines codierend für MD2 bereitgestellt, wobei die TLR4-Sequenz unter Kontrolle des CMV-Promotors und die MD2-Sequenz unter Kontrolle des SV 40-Promotors steht.

Wie sich aus der Figur 8 ergibt, werden z.B. primäre oder immortalisierte humane Fibroblasten 100 stabil mit den Plasmiden für die transgen eingebrachten Reporter- und Rezeptorgene transfiziert und so transgene TLR4- und MD2- Rezeptorgene und transgene SEAP-Reportergene enthaltende Zellen erhalten. Stabil transfizierte Zellen werden in einem Verfahrensschritt 110 selektiert und eine Master-Zellbank (MCB) 115 etabliert. Anschließend wird in an sich bekannter Weise, wie in der EP1 290 145 1 beschrieben, in einem Verfahrensschritt 120 eine dermale Schicht eines dreidimensionalen Hautäquivalenz mit stabil transfizierten immortalisierten oder primären Fibroblasten aufgebaut und in einem Schritt 130 zusätzlich eine epidermale Schicht aus stabil transfizierten Keratinozyten (HaCat-Zell-Linien) aufgebaut.

Der transgen eingebrachte TLR4/MD2-Rezeptorkomplex wird überexprimiert und ermöglicht die Detektion von an diesen Rezeptorkomplex bindenden Substanzen, insbesondere LPS, die nach Bindung an den und anschließender Aktivierung des überexprimierten TLR4/MD2-Rezeptorkomplexes über eine zelleigene Signalkaskade zur Aktivierung des Transkriptionsfaktors NF-κB führen, welcher über die Bindung an die NF-κB-Bindestellen des Reportergenkonstruktes zur Expression der alkalischen Phosphatase (SEAP) führt und damit den Nachweis von Phosphataseaktivität ermöglicht.

Figur 8 zeigt schematisch die Detektion einer zu untersuchenden Substanz, nämlich von LPS, die Überexpression des TLR4/MD2-Rezeptorkomplexes, die nach Bindung von LPS dadurch ermöglichte Transkriptionsaktivierung von NF-κB, dessen Bindung an seine Bindestellen und die Aktivierung der SEAP, welche unmittelbar nachgewiesen werden kann.

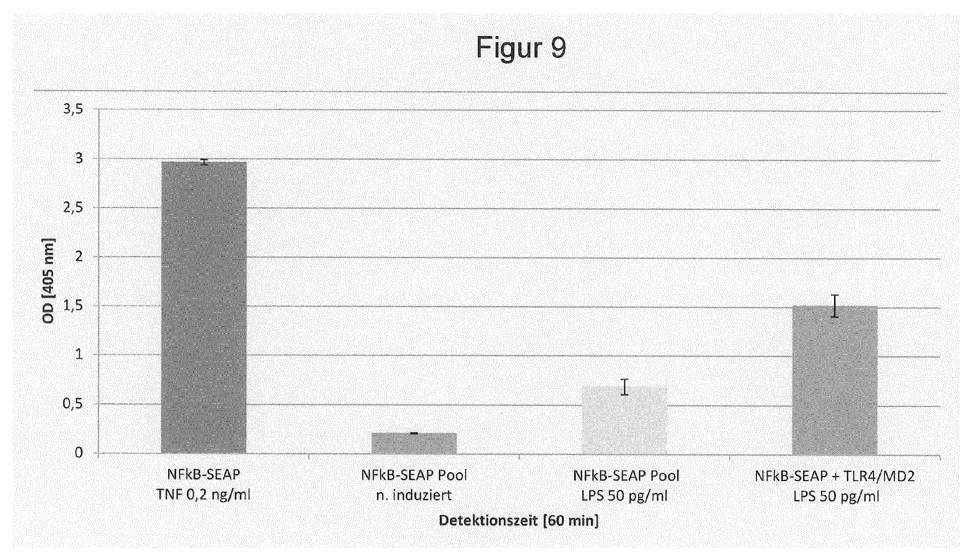

Figur 9 zeigt die erhaltene Signalintensität von primären Fibroblasten-Reporterzellen hergestellt gemäß des vorliegenden Beispiels. Aus der Literatur ist bekannt, dass das Zytokin TNF-α den Transkriptionsfaktor NF-κB aktiviert. TNF wurde daher als Positivkontrolle eingesetzt und zeigt die in Figur 9 dargestellte SEAP-Aktivierung, bewirkt durch TNF-Aktivierung von NF-κB und dessen Bindung an die NF-κBindestellen des stabil transfizierten Reportergen-Konstruktes. Dargestellt ist ferner die SEAP-Aktivität der transfizierten primären Fibroblasten-Reporterzellen ohne und mit LPS-Induktion, und zwar in beiden Fällen ohne Anwesenheit eines transgen transfizierten Rezeptorgens. Die SEAP-Akivität ist im Fall des nicht induzierten stabil transfizierten Reportergenkonstruktes gering, während in LPS induzierten stabil mit dem Reportergen transfizierten Zellen eine signifikante SEAP-Aktivität zu beobachten ist.

Enthalten die stabil mit dem Reportergen transfizierten Zellen darüber hinaus - wie erfindungsgemäß vorgesehen - den transgen eingebrachten TLR4/MD2-Rezeptorkomplex, führt eine LPS-Induktion zu einem sehr deutlichen und signifikanten Anstieg der SEAP-Reporteraktivität, ausgelöst durch die Überexpression des Rezeptorkomplexes und die dadurch ermöglichte quantitativ verbesserte Bindung an LPS. Die erfindungsgemäß bereitgestellten primären Fibroblasten enthaltend das transgene Reportergen codierend SEAP und die transgenen Rezeptorgene (TLR4/MD2-Komplex) ermöglichen daher einen spezifischen signifikanten Nachweis des LPS durch die Expression des Reportergens SEAP.

### Beispiel 4

Erfindungsgemäß ist es auch möglich, immortalisierte Fibroblasten einzusetzen, um das erfindungsgemäße dreidimensionale in vitrohautäquivalent herzustellen.

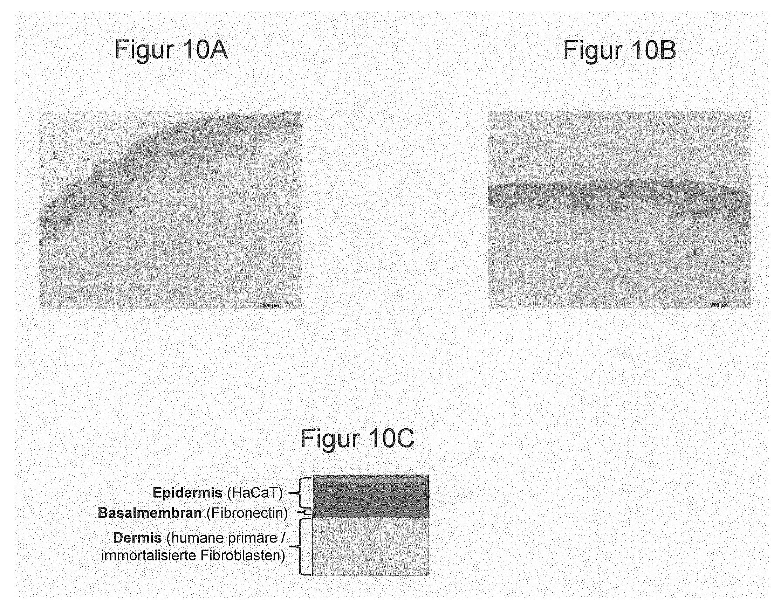

Figur 10a zeigt immortalisierte Fibroblasten mit oberflächlich angeordneten HaCat-Zellen, Figur 10b primäre Fibroblasten mit oberflächlich angeordneten HaCat-Zellen und Figur 10c einen schematischen Aufbau eines dreidimensionalen in vitro-Hautmodells, umfassend eine Epidermis aus HaCat-Zellen, eine Basalmembran (Fibronectin) und eine Dermis aus humanen primären oder immortalisierten Fibroblasten.

## Patentansprüche

1. Dreidimensionales in vitro-Hautäquivalent, umfassend eine Biomatrix und darin eingebettete Zellen mindestens eines ersten Zelltyps, **dadurch gekennzeichnet, dass** die Zellen des ersten Zelltyps (a) mindestens ein transgenes Reportergen, umfassend mindestens eine ein Reporterprotein codierende Nucleotidsequenz unter Kontrolle eines regulatorischen Elements, das durch mindestens einen in dem Zelltyp vorhandenen Transkriptionsfaktor induzierbar ist, und (b) mindestens ein transgenes Rezeptorgen aufweisen, und Zellen eines zweiten Zelltyps, wobei der erste Zelltyp ein Fibroblast und der zweite Zelltyp eine Keratinocyte ist.

2. Hautäquivalent nach Anspruch 1, wobei die Fibroblasten, die Keratinocyten oder beide Zellen einer Zelllinie oder primäre Zellen sind.

3. Hautäquivalent nach einem der vorhergehenden Ansprüche, wobei die Zellen des ersten Zelltyps in der Biomatrix vorwiegend oder allein eine dermale Differenzierungsschicht und die Zellen des zweiten Zelltyps vorwiegend oder allein eine darüberliegende epidermale Differenzierungsschicht ausbilden.

4. Hautäquivalent nach einem der vorhergehenden Ansprüche, wobei auf der epidermalen Differenzierungsschicht eine Hornschicht ausgebildet ist.

5. Hautäquivalent nach einem der vorhergehenden Ansprüche, wobei die Biomatrix eine dreidimensionale, gelartige Kollagenmatrix ist.

6. Hautäquivalent nach einem der vorhergehenden Ansprüche, wobei die ein Reporterprotein codierende Nucleotidsequenz des Reportergens ein fluoreszierendes Protein oder ein Enzym codiert.

7. Hautäquivalent nach einem der vorhergehenden Ansprüche, wobei die ein Reporterprotein codierende Nucleotidsequenz des Reportergens GFP, eGFP, RFP, YFP, alkalische Phosphatase (SEAP), β-Galactosidase, Luciferase oder lacZ codiert.

8. Hautäquivalent nach einem der vorhergehenden Ansprüche, wobei die proteincodierende Nucleotidsequenz des Rezeptorgens Rezeptoren des angeborenen Immunsystems oder Rezeptoren für Wachstumsfaktoren oder Rezeptoren für Cytokine codiert.

9. Hautäquivalent nach einem der vorhergehenden Ansprüche, wobei die Zellen des ersten und zweiten Zelltyps (a) mindestens ein transgenes Reportergen, umfassend mindestens eine ein Reporterprotein codierende Nucleotidsequenz unter Kontrolle eines regulatorischen Elements, das durch mindestens einen in dem Zelltyp vorhandenen Transkriptionsfaktor induzierbar ist und (b) ein transgenes Rezeptorgen aufweisen.

10. Hautäquivalent nach einem der vorhergehenden Ansprüche, wobei in der Biomatrix zusätzlich kein transgenes Reportergen aufweisende primäre Fibroblasten, primäre Keratinozyten oder beide eingebettet sind.

11. Verfahren zur Herstellung eines dreidimensionalen, in vitro-Hautäquivalents nach einem der vorhergehenden Ansprüche, wobei (a) Zellen des ersten Zelltyps und Zellen des zweiten Zelltyps bereitgestellt werden, (b) Zellen dieses ersten Zelltyps mit mindestens einem transgenen Rezeptorgen und mindestens einem transgenen Reportergen transfiziert werden, das mindestens eine ein Reporterprotein codierende Nucleotidsequenz unter Kontrolle eines durch einen in dem Zelltyp vorhandenen Transkriptionsfaktor induzierbaren regulatorischen Elements aufweist, und (c) die Zellen des ersten und zweiten Zelltyps in eine dreidimensionale Biomatrix eingebettet werden und so das Hautäquivalent hergestellt wird.

12. Verfahren nach Anspruch 11, wobei Zellen beider Zelltypen mit mindestens einem transgenen Reportergen transfiziert werden, das mindestens eine ein Reporterprotein codierende Nucleotidsequenz unter Kontrolle eines durch einen in dem Zelltyp vorhandenen Transkriptionsfaktor induzierbaren regulatorischen Elements aufweist und die transfizierten Zellen in der dreidimensionalen Biomatrix eingebettet werden.

13. Verfahren nach einem der vorhergehenden Ansprüche 11 o-der 12, wobei Zellen beider Zelltypen vor der Einbettung in die Biomatrix mit einem transgenen Rezeptorgen transfiziert werden.

14. Verfahren zur Analyse von Substanzen, wobei zu untersuchende Substanzen mit einem Hautäquivalent nach einem der Ansprüche 1 bis 10 in Kontakt gebracht und deren Wirkung auf das Hautäquivalent analysiert wird.

## Claims

1. A three-dimensional in vitro skin equivalent, comprising a biomatrix and cells of at least a first cell type embedded therein, **characterised in that** the cells of the first cell type have (a) at least one transgenic reporter gene, comprising at least one nucleotide sequence encoding for a reporter protein under the control of a regulatory element that is inducible by at least one transcription factor present in the cell type, and (b) at least one transgenic receptor gene, and cells of a second cell type, wherein the first cell type is a fibroblast and the second cell type is a keratinocyte.

2. The skin equivalent according to claim 1, wherein the fibroblasts, the keratinocytes or both are cells of a cell line or primary cells.

3. The skin equivalent according to any one of the preceding claims, wherein in the biomatrix the cells of the first cell type predominantly or exclusively form a dermal differentiation layer, and the cells of the second cell type predominantly or exclusively form an overlaying epidermal differentiation layer.

4. The skin equivalent according to any one of the preceding claims, wherein a corneal layer is formed on the epidermal differentiation layer.

5. The skin equivalent according to any one of the preceding claims, wherein the biomatrix is a three-dimensional gel-like collagen matrix.

6. The skin equivalent according to any one of the preceding claims, wherein die nucleotide sequence of the reporter gene encoding for a reporter protein encodes a fluorescent protein or an enzyme.

7. The skin equivalent according to any one of the preceding claims, wherein the nucleotide sequence of the reporter gene encoding for a reporter protein encodes GFP, eGFP, RFP, YFP, alkaline phosphatase (SEAP), β-galactosidase, luciferase or lacZ.

8. The skin equivalent according to any one of the preceding claims, wherein the protein-encoding nucleotide sequence of the receptor gene encodes for receptors of the innate immune system or for receptors for growth factors or for receptors for cytokines.

9. The skin equivalent according to any one of the preceding claims, wherein the cells of the first and second cell type have (a) at least one transgenic reporter gene comprising at least one nucleotide sequence encoding for a reporter protein under the control of a regulatory element that is inducible by at least one transcription factor present in the cell type and (b) a transgenic receptor gene.

10. The skin equivalent according to any one of the preceding claims, wherein primary fibroblasts or primary keratinocytes, or both, containing no transgenic reporter gene are additionally embedded in the biomatrix.

11. A method for the production of a three-dimensional in vitro skin equivalent according to any one of the preceding claims, wherein (a) cells of the first cell type and cells of the second cell type are provided, (b) cells of this first cell type are transfected with at least one transgenic receptor gene and at least one transgenic reporter gene that has at least one nucleotide sequence encoding for a reporter protein under the control of a regulatory element inducible by a transcription factor present in the cell type, and (c) the cells of the first and the second cell type are embedded in a three-dimensional biomatrix, thus forming the skin equivalent.

12. The method according to claim 11, wherein cells of both cell types are transfected with at least one transgenic reporter gene that has at least one nucleotide sequence encoding for a reporter protein under the control of a regulatory element inducible by a transcription factor present in the cell type, and the transfected cells are embedded in the three-dimensional biomatrix.

13. The method according to one of the preceding claims 11 or 12, wherein cells of both cell types are transfected with a transgenic receptor gene before their embedding in the biomatrix.

14. A method for analysing substances, wherein the substances to be examined are brought into contact with a skin equivalent according to any one of claims 1 to 10, and their effect on the skin equivalent is analysed.

## Revendications

1. Équivalent de peau in vitro et tridimensionnel, comprenant une biomatrice et des cellules d'au moins un premier type de cellule incorporées dans celle-ci, **caractérisé en ce que** les cellules du premier type de cellule présentent (a) au moins un gène rapporteur transgénique comportant au moins une séquence nucléotidique codant pour une protéine rapporteur sous contrôle d'un élément régulateur qui est inductible par au moins un facteur de transcription présent dans ce type de cellule, et (b) au moins un gène récepteur transgénique, et cellules d'un second type de cellule, dans lequel le premier type de cellule est un fibroblaste, et le second type de cellule est un kératinocyte.

2. Équivalent de peau selon la revendication 1, dans lequel les fibroblastes, les kératinocytes ou les deux sont des cellules d'une lignée cellulaire ou des cellules primaires.

3. Équivalent de peau selon l'une quelconque des revendications précédentes, dans lequel, dans la biomatrice, les cellules du premier type de cellule principalement ou uniquement forment une couche de différenciation dermique, et les cellules du second type de cellule principalement ou uniquement forment une couche de différenciation épidermique disposée par-dessus.

4. Équivalent de peau selon l'une quelconque des revendications précédentes, dans lequel une couche cornée est formée sur la couche de différenciation épidermique.

5. Équivalent de peau selon l'une quelconque des revendications précédentes, dans lequel la biomatrice est une matrice tridimensionnelle et gélatiniforme de collagène.

6. Équivalent de peau selon l'une quelconque des revendications précédentes, dans lequel la séquence nucléotidique du gène rapporteur codant pour une protéine rapporteur code pour une protéine fluorescente ou une enzyme.

7. Équivalent de peau selon l'une quelconque des revendications précédentes, dans lequel la séquence nucléotidique du gène rapporteur codant pour une protéine rapporteur code pour GFP, eGFP, RFP, YFP, phosphatase alkalinique (SEAP), β-galactosidase, luciférase ou lacZ.

8. Équivalent de peau selon l'une quelconque des revendications précédentes, dans lequel la séquence nucléotidique du gène récepteur codant pour une protéine code pour des récepteurs du système immunitaire inné ou des récepteurs pour des facteurs de croissance ou des récepteurs pour des cytokines.

9. Équivalent de peau selon l'une quelconque des revendications précédentes, dans lequel les cellules du premier et second type de cellule présentent (a) au moins un gène rapporteur transgénique comportant au moins une séquence nucléotidique codant pour une protéine rapporteur sous contrôle d'un élément régulateur qui est inductible par au moins un facteur de transcription présent dans ce type de cellule et (b) un gène récepteur transgénique.

10. Équivalent de peau selon l'une quelconque des revendications précédentes, dans lequel des fibroblastes primaires ou des kératinocytes primaires ne pas contenant un gène rapporteur transgénique, ou tous les deux, sont additionnellement incorporés dans la biomatrice.

11. Procédé pour la fabrication d'un équivalent de peau in vitro tridimensionnel selon l'une quelconque des revendications précédentes, dans lequel (a) des cellules du premier type de cellule et cellules du second type de cellule sont fournies, (b) des cellules de ce premier type de cellule sont transfectées avec au moins un gène récepteur transgénique et au moins un gène rapporteur transgénique qui présente au moins une séquence nucléotidique codant pour une protéine rapporteur sous le contrôle d'un élément de régulation inductible par un facteur de transcription présent dans ce type cellulaire, et (c) les cellules du premier et second type de cellules sont incorporées dans une biomatrice tridimensionnelle, ainsi formant l'équivalent de peau.

12. Procédé selon la revendication 11, dans lequel des cellules des deux types de cellules sont transfectées avec au moins un gène rapporteur transgénique comportant au moins une séquence nucléotidique codant pour une protéine rapporteur sous contrôle d'un élément régulateur qui est inductible par au moins un facteur de transcription présent dans ce type de cellule, et les cellules transfectées sont incorporées dans la biomatrice tridimensionnelle.

13. Procédé selon l'une quelconque des revendications précédentes 11 ou 12, dans lequel les cellules des deux types de cellules sont transfectées avec un gène récepteur transgénique avant être incorporées dans la biomatrice.

14. Procédé pour l'analyse des substances, dans lequel les substances à examiner sont mises en contact avec un équivalent de peau selon l'une quelconque des revendications 1 à 10, et leur effet sous l'équivalent de peau est analysé.
